# EUROPEAN PATENT SPECIFICATION

(11) **EP 3 490 584 B1**
(45) Date of publication and mention of the grant of the patent: **08.12.2021**
(21) Application number: 17920607.3
(22) Date of filing: 03.08.2017
(51) Int. Cl.: A61K 38/17, A61K 39/39, A61K 39/395, C07K 14/47, C07K 16/30, C07K 19/00

(54) **METHODS AND COMPOSITIONS FOR THE TREATMENT OF MELANOMA**
VERFAHREN UND ZUSAMMENSETZUNGEN ZUR BEHANDLUNG VON MELANOM
MÉTHODES ET COMPOSITIONS PHARMACEUTIQUES DESTINÉES AU TRAITEMENT DU MÉLANOME

(43) Date of publication of application: 05.06.2019
(73) Proprietor: Taiga Biotechnologies, Inc., Aurora, CO 80045 (US)
(72) Inventor: REFAELI, Yosef, Denver 80206 (US); TURNER, Brian C., Denver, Colorado 80246 (US); BIRD, Gregory Alan, Littleton, Colorado 80123 (US)
(74) Representative: Barker Brettell LLP
(86) International application number: PCT/US2017/045336
(87) International publication number: WO 2019/027465

(56) References cited:
- WO-A1-2017/123978
- WO-A2-2011/100477
- US-A1- 2014 109 246
- US-A1- 2014 255 369
- US-A1- 2014 356 392
- US-A1- 2017 044 500

### SEQUENCE LISTING

<110> TAIGA BIOTECHNOLOGIES, INC.
<120> METHODS AND COMPOSITIONS FOR THE TREATMENT OF MELANOMA
<130> 106417-0280
<140> PCT/US2017/045336
   <141> 2017-08-03
<160> 13
<170> PatentIn version 3.5
<210> 1
   <211> 476
   <212> PRT
   <213> Artificial Sequence
<220>
   <223> Description of Artificial Sequence: Synthetic polypeptide
<400> 1
   Met Arg Lys Lys Arg Arg Gln Arg Arg Arg Pro Leu Asn Val Ser Phe
   1               5                   10                  15
   Thr Asn Arg Asn Tyr Asp Leu Asp Tyr Asp Ser Val Gln Pro Tyr Phe
               20                  25                  30
   Tyr Cys Asp Glu Glu Glu Asn Phe Tyr Gln Gln Gln Gln Gln Ser Glu
           35                  40                  45
   Leu Gln Pro Pro Ala Pro Ser Glu Asp Ile Trp Lys Lys Phe Glu Leu
       50                  55                  60
   Leu Pro Thr Pro Pro Leu Ser Pro Ser Arg Arg Ser Gly Leu Cys Ser
   65                  70                  75                  80
   Pro Ser Tyr Val Ala Val Thr Pro Phe Ser Leu Arg Gly Asp Asn Asp
                   85                  90                  95
   Gly Gly Gly Gly Ser Phe Ser Thr Ala Asp Gln Leu Glu Met Val Thr
               100                 105                 110
   Glu Leu Leu Gly Gly Asp Met Val Asn Gln Ser Phe Ile Cys Asp Pro
           115                 120                 125
   Asp Asp Glu Thr Phe Ile Lys Asn Ile Ile Ile Gln Asp Cys Met Trp
       130                 135                 140
   Ser Gly Phe Ser Ala Ala Ala Lys Leu Val Ser Glu Lys Leu Ala Ser
   145                 150                 155                 160
   Tyr Gln Ala Ala Arg Lys Asp Ser Gly Ser Pro Asn Pro Ala Arg Gly
                   165                 170                 175
   His Ser Val Cys Ser Thr Ser Ser Leu Tyr Leu Gln Asp Leu Ser Ala
               180                 185                 190
   Ala Ala Ser Glu Cys Ile Asp Pro Ser Val Val Phe Pro Tyr Pro Leu
           195                 200                 205
   Asn Asp Ser Ser Ser Pro Lys Ser Cys Ala Ser Gln Asp Ser Ser Ala
       210                 215                 220
   Phe Ser Pro Ser Ser Asp Ser Leu Leu Ser Ser Thr Glu Ser Ser Pro
   225                 230                 235                 240
   Gln Gly Ser Pro Glu Pro Leu Val Leu His Glu Glu Thr Pro Pro Thr
                   245                 250                 255
   Thr Ser Ser Asp Ser Glu Glu Glu Gln Glu Asp Glu Glu Glu Ile Asp
               260                 265                 270
   Val Val Ser Val Glu Lys Arg Gln Ala Pro Gly Lys Arg Ser Glu Ser
           275                 280                 285
   Gly Ser Pro Ser Ala Gly Gly His Ser Lys Pro Pro His Ser Pro Leu
       290                 295                 300
   Val Leu Lys Arg Cys His Val Ser Thr His Gln His Asn Tyr Ala Ala
   305                 310                 315                 320
   Pro Pro Ser Thr Arg Lys Asp Tyr Pro Ala Ala Lys Arg Val Lys Leu
                   325                 330                 335
   Asp Ser Val Arg Val Leu Arg Gln Ile Ser Asn Asn Arg Lys Cys Thr
               340                 345                 350
   Ser Pro Arg Ser Ser Asp Thr Glu Glu Asn Val Lys Arg Arg Thr His
           355                 360                 365
   Asn Val Leu Glu Arg Gln Arg Arg Asn Glu Leu Lys Arg Ser Phe Phe
       370                 375                 380
   Ala Leu Arg Asp Gln Ile Pro Glu Leu Glu Asn Asn Glu Lys Ala Pro
   385                 390                 395                 400
   Lys Val Val Ile Leu Lys Lys Ala Thr Ala Tyr Ile Leu Ser Val Gln
                   405                 410                 415
   Ala Glu Glu Gln Lys Leu Ile Ser Glu Glu Asp Leu Leu Arg Lys Arg
               420                 425                 430
   Arg Glu Gln Leu Lys His Lys Leu Glu Gln Leu Arg Lys Gly Glu Leu
           435                 440                 445
   Asn Ser Lys Leu Glu Gly Lys Pro Ile Pro Asn Pro Leu Leu Gly Leu
       450                 455                 460
   Asp Ser Thr Arg Thr Gly His His His His His His
   465                 470                 475
<210> 2
   <211> 439
   <212> PRT
   <213> Homo sapiens
<400> 2
   Met Pro Leu Asn Val Ser Phe Thr Asn Arg Asn Tyr Asp Leu Asp Tyr
   1               5                   10                  15
   Asp Ser Val Gln Pro Tyr Phe Tyr Cys Asp Glu Glu Glu Asn Phe Tyr
               20                  25                  30
   Gln Gln Gln Gln Gln Ser Glu Leu Gln Pro Pro Ala Pro Ser Glu Asp
           35                  40                  45
   Ile Trp Lys Lys Phe Glu Leu Leu Pro Thr Pro Pro Leu Ser Pro Ser
       50                  55                  60
   Arg Arg Ser Gly Leu Cys Ser Pro Ser Tyr Val Ala Val Thr Pro Phe
   65                  70                  75                  80
   Ser Leu Arg Gly Asp Asn Asp Gly Gly Gly Gly Ser Phe Ser Thr Ala
                   85                  90                  95
   Asp Gln Leu Glu Met Val Thr Glu Leu Leu Gly Gly Asp Met Val Asn
               100                 105                 110
   Gln Ser Phe Ile Cys Asp Pro Asp Asp Glu Thr Phe Ile Lys Asn Ile
           115                 120                 125
   Ile Ile Gln Asp Cys Met Trp Ser Gly Phe Ser Ala Ala Ala Lys Leu
       130                 135                 140
   Val Ser Glu Lys Leu Ala Ser Tyr Gln Ala Ala Arg Lys Asp Ser Gly
   145                 150                 155                 160
   Ser Pro Asn Pro Ala Arg Gly His Ser Val Cys Ser Thr Ser Ser Leu
                   165                 170                 175
   Tyr Leu Gln Asp Leu Ser Ala Ala Ala Ser Glu Cys Ile Asp Pro Ser
               180                 185                 190
   Val Val Phe Pro Tyr Pro Leu Asn Asp Ser Ser Ser Pro Lys Ser Cys
           195                 200                 205
   Ala Ser Gln Asp Ser Ser Ala Phe Ser Pro Ser Ser Asp Ser Leu Leu
       210                 215                 220
   Ser Ser Thr Glu Ser Ser Pro Gln Gly Ser Pro Glu Pro Leu Val Leu
   225                 230                 235                 240
   His Glu Glu Thr Pro Pro Thr Thr Ser Ser Asp Ser Glu Glu Glu Gln
                   245                 250                 255
   Glu Asp Glu Glu Glu Ile Asp Val Val Ser Val Glu Lys Arg Gln Ala
               260                 265                 270
   Pro Gly Lys Arg Ser Glu Ser Gly Ser Pro Ser Ala Gly Gly His Ser
           275                 280                 285
   Lys Pro Pro His Ser Pro Leu Val Leu Lys Arg Cys His Val Ser Thr
       290                 295                 300
   His Gln His Asn Tyr Ala Ala Pro Pro Ser Thr Arg Lys Asp Tyr Pro
   305                 310                 315                 320
   Ala Ala Lys Arg Val Lys Leu Asp Ser Val Arg Val Leu Arg Gln Ile
                   325                 330                 335
   Ser Asn Asn Arg Lys Cys Thr Ser Pro Arg Ser Ser Asp Thr Glu Glu
               340                 345                 350
   Asn Val Lys Arg Arg Thr His Asn Val Leu Glu Arg Gln Arg Arg Asn
           355                 360                 365
   Glu Leu Lys Arg Ser Phe Phe Ala Leu Arg Asp Gln Ile Pro Glu Leu
       370                 375                 380
   Glu Asn Asn Glu Lys Ala Pro Lys Val Val Ile Leu Lys Lys Ala Thr
   385                 390                 395                 400
   Ala Tyr Ile Leu Ser Val Gln Ala Glu Glu Gln Lys Leu Ile Ser Glu
   405 410 415
   Glu Asp Leu Leu Arg Lys Arg Arg Glu Gln Leu Lys His Lys Leu Glu
   420 425 430
   Gln Leu Arg Asn Ser Cys Ala
   435
<210> 3
   <211> 450
   <212> PRT
   <213> Homo sapiens
<400> 3
   Met Asp Phe Phe Arg Val Val Glu Asn Gln Gln Pro Pro Ala Thr Met
   1               5                   10                  15
   Pro Leu Asn Val Ser Phe Thr Asn Arg Asn Tyr Asp Leu Asp Tyr Asp
               20                  25                  30
   Ser Val Gln Pro Tyr Phe Tyr Cys Asp Glu Glu Glu Asn Phe Tyr Gln
           35                  40                  45
   Gln Gln Gln Gln Ser Glu Leu Gln Pro Pro Ala Pro Ser Glu Asp Ile
       50                  55                  60
   Trp Lys Lys Phe Glu Leu Leu Pro Thr Pro Pro Leu Ser Pro Ser Arg
   65                  70                  75                  80
   Arg Ser Gly Leu Cys Ser Pro Ser Tyr Val Ala Val Thr Pro Phe Ser
                   85                  90                  95
   Leu Arg Gly Asp Asn Asp Gly Gly Gly Gly Ser Phe Ser Thr Ala Asp
               100                 105                 110
   Gln Leu Glu Met Val Thr Glu Leu Leu Gly Gly Asp Met Val Asn Gln
           115                 120                 125
   Ser Phe Ile Cys Asp Pro Asp Asp Glu Thr Phe Ile Lys Asn Ile Ile
       130                 135                 140
   Ile Gln Asp Cys Met Trp Ser Gly Phe Ser Ala Ala Ala Lys Leu Val
   145                 150                 155                 160
   Ser Glu Lys Leu Ala Ser Tyr Gln Ala Ala Arg Lys Asp Ser Gly Ser
                   165                 170                 175
   Pro Asn Pro Ala Arg Gly His Ser Val Cys Ser Thr Ser Ser Leu Tyr
               180                 185                 190
   Leu Gln Asp Leu Ser Ala Ala Ala Ser Glu Cys Ile Asp Pro Ser Val
           195                 200                 205
   Val Phe Pro Tyr Pro Leu Asn Asp Ser Ser Ser Pro Lys Ser Cys Ala
       210                 215                 220
   Ser Gln Asp Ser Ser Ala Phe Ser Pro Ser Ser Asp Ser Leu Leu Ser
   225                 230                 235                 240
   Ser Thr Glu Ser Ser Pro Gln Gly Ser Pro Glu Pro Leu Val Leu His
                   245                 250                 255
   Glu Glu Thr Pro Pro Thr Thr Ser Ser Asp Ser Glu Glu Glu Gln Glu
               260                 265                 270
   Asp Glu Glu Glu Ile Asp Val Val Ser Val Glu Lys Arg Gln Ala Pro
           275                 280                 285
   Gly Lys Arg Ser Glu Ser Gly Ser Pro Ser Ala Gly Gly His Ser Lys
       290                 295                 300
   Pro Pro His Ser Pro Leu Val Leu Lys Arg Cys His Val Ser Thr His
   305                 310                 315                 320
   Gln His Asn Tyr Ala Ala Pro Pro Ser Thr Arg Lys Asp Tyr Pro Ala
                   325                 330                 335
   Ala Lys Arg Val Lys Leu Asp Ser Val Arg Val Leu Arg Gln Ile Ser
               340                 345                 350
   Asn Asn Arg Lys Cys Thr Ser Pro Arg Ser Ser Asp Thr Glu Glu Asn
           355                 360                 365
   Val Lys Arg Arg Thr His Asn Val Leu Glu Arg Gln Arg Arg Asn Glu
       370                 375                 380
   Leu Lys Arg Ser Phe Phe Ala Leu Arg Asp Gln Ile Pro Glu Leu Glu
   385                 390                 395                 400
   Asn Asn Glu Lys Ala Pro Lys Val Val Ile Leu Lys Lys Ala Thr Ala
                   405                 410                 415
   Tyr Ile Leu Ser Val Gln Ala Glu Glu Gln Lys Leu Ile Ser Glu Glu
               420                 425                 430
   Asp Leu Leu Arg Lys Arg Arg Glu Gln Leu Lys His Lys Leu Glu Gln
   435 440 445
   Leu Arg
   450
<210> 4
   <211> 434
   <212> PRT
   <213> Homo sapiens
<400> 4
   Pro Leu Asn Val Ser Phe Thr Asn Arg Asn Tyr Asp Leu Asp Tyr Asp
   1               5                   10                  15
   Ser Val Gln Pro Tyr Phe Tyr Cys Asp Glu Glu Glu Asn Phe Tyr Gln
               20                  25                  30
   Gln Gln Gln Gln Ser Glu Leu Gln Pro Pro Ala Pro Ser Glu Asp Ile
           35                  40                  45
   Trp Lys Lys Phe Glu Leu Leu Pro Thr Pro Pro Leu Ser Pro Ser Arg
       50                  55                  60
   Arg Ser Gly Leu Cys Ser Pro Ser Tyr Val Ala Val Thr Pro Phe Ser
   65                  70                  75                  80
   Leu Arg Gly Asp Asn Asp Gly Gly Gly Gly Ser Phe Ser Thr Ala Asp
                   85                  90                  95
   Gln Leu Glu Met Val Thr Glu Leu Leu Gly Gly Asp Met Val Asn Gln
               100                 105                 110
   Ser Phe Ile Cys Asp Pro Asp Asp Glu Thr Phe Ile Lys Asn Ile Ile
           115                 120                 125
   Ile Gln Asp Cys Met Trp Ser Gly Phe Ser Ala Ala Ala Lys Leu Val
       130                 135                 140
   Ser Glu Lys Leu Ala Ser Tyr Gln Ala Ala Arg Lys Asp Ser Gly Ser
   145                 150                 155                 160
   Pro Asn Pro Ala Arg Gly His Ser Val Cys Ser Thr Ser Ser Leu Tyr
                   165                 170                 175
   Leu Gln Asp Leu Ser Ala Ala Ala Ser Glu Cys Ile Asp Pro Ser Val
               180                 185                 190
   Val Phe Pro Tyr Pro Leu Asn Asp Ser Ser Ser Pro Lys Ser Cys Ala
           195                 200                 205
   Ser Gln Asp Ser Ser Ala Phe Ser Pro Ser Ser Asp Ser Leu Leu Ser
       210                 215                 220
   Ser Thr Glu Ser Ser Pro Gln Gly Ser Pro Glu Pro Leu Val Leu His
   225                 230                 235                 240
   Glu Glu Thr Pro Pro Thr Thr Ser Ser Asp Ser Glu Glu Glu Gln Glu
                   245                 250                 255
   Asp Glu Glu Glu Ile Asp Val Val Ser Val Glu Lys Arg Gln Ala Pro
               260                 265                 270
   Gly Lys Arg Ser Glu Ser Gly Ser Pro Ser Ala Gly Gly His Ser Lys
           275                 280                 285
   Pro Pro His Ser Pro Leu Val Leu Lys Arg Cys His Val Ser Thr His
       290                 295                 300
   Gln His Asn Tyr Ala Ala Pro Pro Ser Thr Arg Lys Asp Tyr Pro Ala
   305                 310                 315                 320
   Ala Lys Arg Val Lys Leu Asp Ser Val Arg Val Leu Arg Gln Ile Ser
                   325                 330                 335
   Asn Asn Arg Lys Cys Thr Ser Pro Arg Ser Ser Asp Thr Glu Glu Asn
               340                 345                 350
   Val Lys Arg Arg Thr His Asn Val Leu Glu Arg Gln Arg Arg Asn Glu
           355                 360                 365
   Leu Lys Arg Ser Phe Phe Ala Leu Arg Asp Gln Ile Pro Glu Leu Glu
       370                 375                 380
   Asn Asn Glu Lys Ala Pro Lys Val Val Ile Leu Lys Lys Ala Thr Ala
   385                 390                 395                 400
   Tyr Ile Leu Ser Val Gln Ala Glu Glu Gln Lys Leu Ile Ser Glu Glu
                   405                 410                 415
   Asp Leu Leu Arg Lys Arg Arg Glu Gln Leu Lys His Lys Leu Glu Gln
               420                 425                 430
   Leu Arg
<210> 5
   <211> 67
   <212> PRT
   <213> Homo sapiens
<400> 5
   Glu Leu Lys Arg Ser Phe Phe Ala Leu Arg Asp Gln Ile Pro Glu Leu
   1               5                   10                  15
   Glu Asn Asn Glu Lys Ala Pro Lys Val Val Ile Leu Lys Lys Ala Thr
               20                  25                  30
   Ala Tyr Ile Leu Ser Val Gln Ala Glu Glu Gln Lys Leu Ile Ser Glu
           35                  40                  45
   Glu Asp Leu Leu Arg Lys Arg Arg Glu Gln Leu Lys His Lys Leu Glu
       50                  55                  60
   Gln Leu Arg
   65
<210> 6
   <211> 14
   <212> PRT
   <213> Unknown
<220>
   <223> Description of Unknown: E-box DNA binding domain sequence
<400> 6
   Lys Arg Arg Thr His Asn Val Leu Glu Arg Gln Arg Arg Asn
   1               5                   10
<210> 7
   <211> 10
   <212> PRT
   <213> Human immunodeficiency virus 1
<400> 7
   Met Arg Lys Lys Arg Arg Gln Arg Arg Arg
   1               5                   10
<210> 8
   <211> 6
   <212> PRT
   <213> Artificial Sequence
<220>
   <223> Description of Artificial Sequence: Synthetic 6xHis tag
<400> 8
   His His His His His His
   1               5
<210> 9
   <211> 14
   <212> PRT
   <213> Artificial Sequence
<220>
   <223> Description of Artificial Sequence: Synthetic peptide
<400> 9
   Gly Lys Pro Ile Pro Asn Pro Leu Leu Gly Leu Asp Ser Thr
   1               5                   10
<210> 10
   <211> 9
   <212> PRT
   <213> Artificial Sequence
<220>
   <223> Description of Artificial Sequence: Synthetic peptide
<400> 10
   Ile Pro Asn Pro Leu Leu Gly Leu Asp
   1               5
<210> 11
   <211> 454
   <212> PRT
   <213> Homo sapiens
<400> 11
   Met Asp Phe Phe Arg Val Val Glu Asn Gln Gln Pro Pro Ala Thr Met
   1               5                   10                  15
   Pro Leu Asn Val Ser Phe Thr Asn Arg Asn Tyr Asp Leu Asp Tyr Asp
               20                  25                  30
   Ser Val Gln Pro Tyr Phe Tyr Cys Asp Glu Glu Glu Asn Phe Tyr Gln
           35                  40                  45
   Gln Gln Gln Gln Ser Glu Leu Gln Pro Pro Ala Pro Ser Glu Asp Ile
       50                  55                  60
   Trp Lys Lys Phe Glu Leu Leu Pro Thr Pro Pro Leu Ser Pro Ser Arg
   65                  70                  75                  80
   Arg Ser Gly Leu Cys Ser Pro Ser Tyr Val Ala Val Thr Pro Phe Ser
                   85                  90                  95
   Leu Arg Gly Asp Asn Asp Gly Gly Gly Gly Ser Phe Ser Thr Ala Asp
               100                 105                 110
   Gln Leu Glu Met Val Thr Glu Leu Leu Gly Gly Asp Met Val Asn Gln
           115                 120                 125
   Ser Phe Ile Cys Asp Pro Asp Asp Glu Thr Phe Ile Lys Asn Ile Ile
       130                 135                 140
   Ile Gln Asp Cys Met Trp Ser Gly Phe Ser Ala Ala Ala Lys Leu Val
   145                 150                 155                 160
   Ser Glu Lys Leu Ala Ser Tyr Gln Ala Ala Arg Lys Asp Ser Gly Ser
                   165                 170                 175
   Pro Asn Pro Ala Arg Gly His Ser Val Cys Ser Thr Ser Ser Leu Tyr
               180                 185                 190
   Leu Gln Asp Leu Ser Ala Ala Ala Ser Glu Cys Ile Asp Pro Ser Val
           195                 200                 205
   Val Phe Pro Tyr Pro Leu Asn Asp Ser Ser Ser Pro Lys Ser Cys Ala
       210                 215                 220
   Ser Gln Asp Ser Ser Ala Phe Ser Pro Ser Ser Asp Ser Leu Leu Ser
   225                 230                 235                 240
   Ser Thr Glu Ser Ser Pro Gln Gly Ser Pro Glu Pro Leu Val Leu His
                   245                 250                 255
   Glu Glu Thr Pro Pro Thr Thr Ser Ser Asp Ser Glu Glu Glu Gln Glu
               260                 265                 270
   Asp Glu Glu Glu Ile Asp Val Val Ser Val Glu Lys Arg Gln Ala Pro
           275                 280                 285
   Gly Lys Arg Ser Glu Ser Gly Ser Pro Ser Ala Gly Gly His Ser Lys
       290                 295                 300
   Pro Pro His Ser Pro Leu Val Leu Lys Arg Cys His Val Ser Thr His
   305                 310                 315                 320
   Gln His Asn Tyr Ala Ala Pro Pro Ser Thr Arg Lys Asp Tyr Pro Ala
                   325                 330                 335
   Ala Lys Arg Val Lys Leu Asp Ser Val Arg Val Leu Arg Gln Ile Ser
               340                 345                 350
   Asn Asn Arg Lys Cys Thr Ser Pro Arg Ser Ser Asp Thr Glu Glu Asn
           355                 360                 365
   Val Lys Arg Arg Thr His Asn Val Leu Glu Arg Gln Arg Arg Asn Glu
   370 375 380
   Leu Lys Arg Ser Phe Phe Ala Leu Arg Asp Gln Ile Pro Glu Leu Glu
   385 390 395 400
   Asn Asn Glu Lys Ala Pro Lys Val Val Ile Leu Lys Lys Ala Thr Ala
   405 410 415
   Tyr Ile Leu Ser Val Gln Ala Glu Glu Gln Lys Leu Ile Ser Glu Glu
   420 425 430
   Asp Leu Leu Arg Lys Arg Arg Glu Gln Leu Lys His Lys Leu Glu Gln
   435 440 445
   Leu Arg Asn Ser Cys Ala
   450
<210> 12
   <211> 9
   <212> PRT
   <213> Artificial Sequence
<220>
   <223> Description of Artificial Sequence: Synthetic peptide
<400> 12
   Lys Gly Glu Leu Asn Ser Lys Leu Glu
   1 5
<210> 13
   <211> 9
   <212> PRT
   <213> Human immunodeficiency virus 1
<400> 13 SEQUENCE LISTING
<110> TAIGA BIOTECHNOLOGIES, INC.
<120> METHODS AND COMPOSITIONS FOR THE TREATMENT OF MELANOMA
<130> JA99006P.EPP
<140> EP17920607.3
   <141> 2017-08-03
<150> PCT/US2017/045336
   <151> 2017-08-03
<160> 13
<170> PatentIn version 3.5
<210> 1
   <211> 476
   <212> PRT
   <213> Artificial Sequence
<220>
   <223> Description of Artificial Sequence: Synthetic polypeptide
<400> 1
   Met Arg Lys Lys Arg Arg Gln Arg Arg Arg Pro Leu Asn Val Ser Phe
   1               5                   10                  15
   Thr Asn Arg Asn Tyr Asp Leu Asp Tyr Asp Ser Val Gln Pro Tyr Phe
               20                  25                  30
   Tyr Cys Asp Glu Glu Glu Asn Phe Tyr Gln Gln Gln Gln Gln Ser Glu
           35                  40                  45
   Leu Gln Pro Pro Ala Pro Ser Glu Asp Ile Trp Lys Lys Phe Glu Leu
       50                  55                  60
   Leu Pro Thr Pro Pro Leu Ser Pro Ser Arg Arg Ser Gly Leu Cys Ser
   65                  70                  75                  80
   Pro Ser Tyr Val Ala Val Thr Pro Phe Ser Leu Arg Gly Asp Asn Asp
                   85                  90                  95
   Gly Gly Gly Gly Ser Phe Ser Thr Ala Asp Gln Leu Glu Met Val Thr
               100                 105                 110
   Glu Leu Leu Gly Gly Asp Met Val Asn Gln Ser Phe Ile Cys Asp Pro
           115                 120                 125
   Asp Asp Glu Thr Phe Ile Lys Asn Ile Ile Ile Gln Asp Cys Met Trp
       130                 135                 140
   Ser Gly Phe Ser Ala Ala Ala Lys Leu Val Ser Glu Lys Leu Ala Ser
   145                 150                 155                 160
   Tyr Gln Ala Ala Arg Lys Asp Ser Gly Ser Pro Asn Pro Ala Arg Gly
                   165                 170                 175
   His Ser Val Cys Ser Thr Ser Ser Leu Tyr Leu Gln Asp Leu Ser Ala
               180                 185                 190
   Ala Ala Ser Glu Cys Ile Asp Pro Ser Val Val Phe Pro Tyr Pro Leu
           195                 200                 205
   Asn Asp Ser Ser Ser Pro Lys Ser Cys Ala Ser Gln Asp Ser Ser Ala
       210                 215                 220
   Phe Ser Pro Ser Ser Asp Ser Leu Leu Ser Ser Thr Glu Ser Ser Pro
   225                 230                 235                 240
   Gln Gly Ser Pro Glu Pro Leu Val Leu His Glu Glu Thr Pro Pro Thr
                   245                 250                 255
   Thr Ser Ser Asp Ser Glu Glu Glu Gln Glu Asp Glu Glu Glu Ile Asp
               260                 265                 270
   Val Val Ser Val Glu Lys Arg Gln Ala Pro Gly Lys Arg Ser Glu Ser
           275                 280                 285
   Gly Ser Pro Ser Ala Gly Gly His Ser Lys Pro Pro His Ser Pro Leu
       290                 295                 300
   Val Leu Lys Arg Cys His Val Ser Thr His Gln His Asn Tyr Ala Ala
   305                 310                 315                 320
   Pro Pro Ser Thr Arg Lys Asp Tyr Pro Ala Ala Lys Arg Val Lys Leu
                   325                 330                 335
   Asp Ser Val Arg Val Leu Arg Gln Ile Ser Asn Asn Arg Lys Cys Thr
               340                 345                 350
   Ser Pro Arg Ser Ser Asp Thr Glu Glu Asn Val Lys Arg Arg Thr His
           355                 360                 365
   Asn Val Leu Glu Arg Gln Arg Arg Asn Glu Leu Lys Arg Ser Phe Phe
       370                 375                 380
   Ala Leu Arg Asp Gln Ile Pro Glu Leu Glu Asn Asn Glu Lys Ala Pro
   385                 390                 395                 400
   Lys Val Val Ile Leu Lys Lys Ala Thr Ala Tyr Ile Leu Ser Val Gln
                   405                 410                 415
   Ala Glu Glu Gln Lys Leu Ile Ser Glu Glu Asp Leu Leu Arg Lys Arg
               420                 425                 430
   Arg Glu Gln Leu Lys His Lys Leu Glu Gln Leu Arg Lys Gly Glu Leu
           435                 440                 445
   Asn Ser Lys Leu Glu Gly Lys Pro Ile Pro Asn Pro Leu Leu Gly Leu
       450                 455                 460
   Asp Ser Thr Arg Thr Gly His His His His His His
   465                 470                 475
<210> 2
   <211> 439
   <212> PRT
   <213> Homo sapiens
<400> 2
   Met Pro Leu Asn Val Ser Phe Thr Asn Arg Asn Tyr Asp Leu Asp Tyr
   1               5                   10                  15
   Asp Ser Val Gln Pro Tyr Phe Tyr Cys Asp Glu Glu Glu Asn Phe Tyr
               20                  25                  30
   Gln Gln Gln Gln Gln Ser Glu Leu Gln Pro Pro Ala Pro Ser Glu Asp
           35                  40                  45
   Ile Trp Lys Lys Phe Glu Leu Leu Pro Thr Pro Pro Leu Ser Pro Ser
       50                  55                  60
   Arg Arg Ser Gly Leu Cys Ser Pro Ser Tyr Val Ala Val Thr Pro Phe
   65                  70                  75                  80
   Ser Leu Arg Gly Asp Asn Asp Gly Gly Gly Gly Ser Phe Ser Thr Ala
                   85                  90                  95
   Asp Gln Leu Glu Met Val Thr Glu Leu Leu Gly Gly Asp Met Val Asn
               100                 105                 110
   Gln Ser Phe Ile Cys Asp Pro Asp Asp Glu Thr Phe Ile Lys Asn Ile
           115                 120                 125
   Ile Ile Gln Asp Cys Met Trp Ser Gly Phe Ser Ala Ala Ala Lys Leu
       130                 135                 140
   Val Ser Glu Lys Leu Ala Ser Tyr Gln Ala Ala Arg Lys Asp Ser Gly
   145                 150                 155                 160
   Ser Pro Asn Pro Ala Arg Gly His Ser Val Cys Ser Thr Ser Ser Leu
                   165                 170                 175
   Tyr Leu Gln Asp Leu Ser Ala Ala Ala Ser Glu Cys Ile Asp Pro Ser
               180                 185                 190
   Val Val Phe Pro Tyr Pro Leu Asn Asp Ser Ser Ser Pro Lys Ser Cys
           195                 200                 205
   Ala Ser Gln Asp Ser Ser Ala Phe Ser Pro Ser Ser Asp Ser Leu Leu
       210                 215                 220
   Ser Ser Thr Glu Ser Ser Pro Gln Gly Ser Pro Glu Pro Leu Val Leu
   225                 230                 235                 240
   His Glu Glu Thr Pro Pro Thr Thr Ser Ser Asp Ser Glu Glu Glu Gln
                   245                 250                 255
   Glu Asp Glu Glu Glu Ile Asp Val Val Ser Val Glu Lys Arg Gln Ala
               260                 265                 270
   Pro Gly Lys Arg Ser Glu Ser Gly Ser Pro Ser Ala Gly Gly His Ser
           275                 280                 285
   Lys Pro Pro His Ser Pro Leu Val Leu Lys Arg Cys His Val Ser Thr
       290                 295                 300
   His Gln His Asn Tyr Ala Ala Pro Pro Ser Thr Arg Lys Asp Tyr Pro
   305                 310                 315                 320
   Ala Ala Lys Arg Val Lys Leu Asp Ser Val Arg Val Leu Arg Gln Ile
                   325                 330                 335
   Ser Asn Asn Arg Lys Cys Thr Ser Pro Arg Ser Ser Asp Thr Glu Glu
               340                 345                 350
   Asn Val Lys Arg Arg Thr His Asn Val Leu Glu Arg Gln Arg Arg Asn
           355                 360                 365
   Glu Leu Lys Arg Ser Phe Phe Ala Leu Arg Asp Gln Ile Pro Glu Leu
       370                 375                 380
   Glu Asn Asn Glu Lys Ala Pro Lys Val Val Ile Leu Lys Lys Ala Thr
   385                 390                 395                 400
   Ala Tyr Ile Leu Ser Val Gln Ala Glu Glu Gln Lys Leu Ile Ser Glu
   405 410 415
   Glu Asp Leu Leu Arg Lys Arg Arg Glu Gln Leu Lys His Lys Leu Glu
   420 425 430
   Gln Leu Arg Asn Ser Cys Ala
   435
<210> 3
   <211> 450
   <212> PRT
   <213> Homo sapiens
<400> 3 Leu Arg
   450
<210> 4
   <211> 434
   <212> PRT
   <213> Homo sapiens
<400> 4
   Pro Leu Asn Val Ser Phe Thr Asn Arg Asn Tyr Asp Leu Asp Tyr Asp
   1               5                   10                  15
   Ser Val Gln Pro Tyr Phe Tyr Cys Asp Glu Glu Glu Asn Phe Tyr Gln
               20                  25                  30
   Gln Gln Gln Gln Ser Glu Leu Gln Pro Pro Ala Pro Ser Glu Asp Ile
           35                  40                  45
   Trp Lys Lys Phe Glu Leu Leu Pro Thr Pro Pro Leu Ser Pro Ser Arg
       50                  55                  60
   Arg Ser Gly Leu Cys Ser Pro Ser Tyr Val Ala Val Thr Pro Phe Ser
   65                  70                  75                  80
   Leu Arg Gly Asp Asn Asp Gly Gly Gly Gly Ser Phe Ser Thr Ala Asp
                   85                  90                  95
   Gln Leu Glu Met Val Thr Glu Leu Leu Gly Gly Asp Met Val Asn Gln
               100                 105                 110
   Ser Phe Ile Cys Asp Pro Asp Asp Glu Thr Phe Ile Lys Asn Ile Ile
           115                 120                 125
   Ile Gln Asp Cys Met Trp Ser Gly Phe Ser Ala Ala Ala Lys Leu Val
       130                 135                 140
   Ser Glu Lys Leu Ala Ser Tyr Gln Ala Ala Arg Lys Asp Ser Gly Ser
   145                 150                 155                 160
   Pro Asn Pro Ala Arg Gly His Ser Val Cys Ser Thr Ser Ser Leu Tyr
                   165                 170                 175
   Leu Gln Asp Leu Ser Ala Ala Ala Ser Glu Cys Ile Asp Pro Ser Val
               180                 185                 190
   Val Phe Pro Tyr Pro Leu Asn Asp Ser Ser Ser Pro Lys Ser Cys Ala
           195                 200                 205
   Ser Gln Asp Ser Ser Ala Phe Ser Pro Ser Ser Asp Ser Leu Leu Ser
       210                 215                 220
   Ser Thr Glu Ser Ser Pro Gln Gly Ser Pro Glu Pro Leu Val Leu His
   225                 230                 235                 240
   Glu Glu Thr Pro Pro Thr Thr Ser Ser Asp Ser Glu Glu Glu Gln Glu
                   245                 250                 255
   Asp Glu Glu Glu Ile Asp Val Val Ser Val Glu Lys Arg Gln Ala Pro
               260                 265                 270
   Gly Lys Arg Ser Glu Ser Gly Ser Pro Ser Ala Gly Gly His Ser Lys
           275                 280                 285
   Pro Pro His Ser Pro Leu Val Leu Lys Arg Cys His Val Ser Thr His
       290                 295                 300
   Gln His Asn Tyr Ala Ala Pro Pro Ser Thr Arg Lys Asp Tyr Pro Ala
   305                 310                 315                 320
   Ala Lys Arg Val Lys Leu Asp Ser Val Arg Val Leu Arg Gln Ile Ser
                   325                 330                 335
   Asn Asn Arg Lys Cys Thr Ser Pro Arg Ser Ser Asp Thr Glu Glu Asn
               340                 345                 350
   Val Lys Arg Arg Thr His Asn Val Leu Glu Arg Gln Arg Arg Asn Glu
           355                 360                 365
   Leu Lys Arg Ser Phe Phe Ala Leu Arg Asp Gln Ile Pro Glu Leu Glu
       370                 375                 380
   Asn Asn Glu Lys Ala Pro Lys Val Val Ile Leu Lys Lys Ala Thr Ala
   385                 390                 395                 400
   Tyr Ile Leu Ser Val Gln Ala Glu Glu Gln Lys Leu Ile Ser Glu Glu
                   405                 410                 415
   Asp Leu Leu Arg Lys Arg Arg Glu Gln Leu Lys His Lys Leu Glu Gln
               420                 425                 430
   Leu Arg
<210> 5
   <211> 67
   <212> PRT
   <213> Homo sapiens
<400> 5
   Glu Leu Lys Arg Ser Phe Phe Ala Leu Arg Asp Gln Ile Pro Glu Leu
   1               5                   10                  15
   Glu Asn Asn Glu Lys Ala Pro Lys Val Val Ile Leu Lys Lys Ala Thr
               20                  25                  30
   Ala Tyr Ile Leu Ser Val Gln Ala Glu Glu Gln Lys Leu Ile Ser Glu
           35                  40                  45
   Glu Asp Leu Leu Arg Lys Arg Arg Glu Gln Leu Lys His Lys Leu Glu
       50                  55                  60
   Gln Leu Arg
   65
<210> 6
   <211> 14
   <212> PRT
   <213> Unknown
<220>
   <223> Description of Unknown: E-box DNA binding domain sequence
<400> 6
   Lys Arg Arg Thr His Asn Val Leu Glu Arg Gln Arg Arg Asn
   1               5                   10
<210> 7
   <211> 10
   <212> PRT
   <213> Human immunodeficiency virus 1
<400> 7
   Met Arg Lys Lys Arg Arg Gln Arg Arg Arg
   1               5                   10
<210> 8
   <211> 6
   <212> PRT
   <213> Artificial Sequence
<220>
   Description of Artificial Sequence: Synthetic 6xHis tag
<400> 8
   His His His His His His
   1 5
<210> 9
   <211> 14
   <212> PRT
   <213> Artificial Sequence
<220>
   <223> Description of Artificial Sequence: Synthetic peptide
<400> 9
   Gly Lys Pro Ile Pro Asn Pro Leu Leu Gly Leu Asp Ser Thr
   1 5 10
<210> 10
   <211> 9
   <212> PRT
   <213> Artificial Sequence
<220>
   <223> Description of Artificial Sequence: Synthetic peptide
<400> 10
   Ile Pro Asn Pro Leu Leu Gly Leu Asp
   1 5
<210> 11
   <211> 454
   <212> PRT
   <213> Homo sapiens
<400> 11
   Met Asp Phe Phe Arg Val Val Glu Asn Gln Gln Pro Pro Ala Thr Met
   1               5                   10                  15
   Pro Leu Asn Val Ser Phe Thr Asn Arg Asn Tyr Asp Leu Asp Tyr Asp
               20                  25                  30
   Ser Val Gln Pro Tyr Phe Tyr Cys Asp Glu Glu Glu Asn Phe Tyr Gln
           35                  40                  45
   Gln Gln Gln Gln Ser Glu Leu Gln Pro Pro Ala Pro Ser Glu Asp Ile
       50                  55                  60
   Trp Lys Lys Phe Glu Leu Leu Pro Thr Pro Pro Leu Ser Pro Ser Arg
   65                  70                  75                  80
   Arg Ser Gly Leu Cys Ser Pro Ser Tyr Val Ala Val Thr Pro Phe Ser
                   85                  90                  95
   Leu Arg Gly Asp Asn Asp Gly Gly Gly Gly Ser Phe Ser Thr Ala Asp
               100                 105                 110
   Gln Leu Glu Met Val Thr Glu Leu Leu Gly Gly Asp Met Val Asn Gln
           115                 120                 125
   Ser Phe Ile Cys Asp Pro Asp Asp Glu Thr Phe Ile Lys Asn Ile Ile
       130                 135                 140
   Ile Gln Asp Cys Met Trp Ser Gly Phe Ser Ala Ala Ala Lys Leu Val
   145                 150                 155                 160
   Ser Glu Lys Leu Ala Ser Tyr Gln Ala Ala Arg Lys Asp Ser Gly Ser
                   165                 170                 175
   Pro Asn Pro Ala Arg Gly His Ser Val Cys Ser Thr Ser Ser Leu Tyr
               180                 185                 190
   Leu Gln Asp Leu Ser Ala Ala Ala Ser Glu Cys Ile Asp Pro Ser Val
           195                 200                 205
   Val Phe Pro Tyr Pro Leu Asn Asp Ser Ser Ser Pro Lys Ser Cys Ala
       210                 215                 220
   Ser Gln Asp Ser Ser Ala Phe Ser Pro Ser Ser Asp Ser Leu Leu Ser
   225                 230                 235                 240
   Ser Thr Glu Ser Ser Pro Gln Gly Ser Pro Glu Pro Leu Val Leu His
                   245                 250                 255
   Glu Glu Thr Pro Pro Thr Thr Ser Ser Asp Ser Glu Glu Glu Gln Glu
               260                 265                 270
   Asp Glu Glu Glu Ile Asp Val Val Ser Val Glu Lys Arg Gln Ala Pro
           275                 280                 285
   Gly Lys Arg Ser Glu Ser Gly Ser Pro Ser Ala Gly Gly His Ser Lys
       290                 295                 300
   Pro Pro His Ser Pro Leu Val Leu Lys Arg Cys His Val Ser Thr His
   305                 310                 315                 320
   Gln His Asn Tyr Ala Ala Pro Pro Ser Thr Arg Lys Asp Tyr Pro Ala
                   325                 330                 335
   Ala Lys Arg Val Lys Leu Asp Ser Val Arg Val Leu Arg Gln Ile Ser
               340                 345                 350
   Asn Asn Arg Lys Cys Thr Ser Pro Arg Ser Ser Asp Thr Glu Glu Asn
                   355                 360                 365
   Val Lys Arg Arg Thr His Asn Val Leu Glu Arg Gln Arg Arg Asn Glu
       370                 375                 380
   Leu Lys Arg Ser Phe Phe Ala Leu Arg Asp Gln Ile Pro Glu Leu Glu
   385                 390                 395                 400
   Asn Asn Glu Lys Ala Pro Lys Val Val Ile Leu Lys Lys Ala Thr Ala
                   405                 410                 415
   Tyr Ile Leu Ser Val Gln Ala Glu Glu Gln Lys Leu Ile Ser Glu Glu
               420                 425                 430
   Asp Leu Leu Arg Lys Arg Arg Glu Gln Leu Lys His Lys Leu Glu Gln
           435                 440                 445
   Leu Arg Asn Ser Cys Ala
       450
<210> 12
   <211> 9
   <212> PRT
   <213> Artificial Sequence
<220>
   <223> Description of Artificial Sequence: Synthetic peptide
<400> 12
   Lys Gly Glu Leu Asn Ser Lys Leu Glu
   1               5
<210> 13
   <211> 9
   <212> PRT
   <213> Human immunodeficiency virus 1
<400> 13
   Arg Lys Lys Arg Arg Gln Arg Arg Arg
   1               5

## Description

### BACKGROUND OF THE INVENTION

Adoptive cell transfer (ACT) is a form of immunotherapy that involves the transfer of immune cells with antitumor activity into patients. ACT typically involves isolation of lymphocytes with antitumor activity from a patient, culturing the lymphocytes *in vitro* to expand the population, and then infusing the lymphocytes into the cancer-bearing host. Lymphocytes used for adoptive transfer can either be derived from the stroma of resected tumors (e.g., tumor infiltrating lymphocytes), from the lymphatics or lymph nodes, or from the blood. In some cases, the isolated lymphocytes are genetically engineered to express antitumor T cell receptors (TCRs) or chimeric antigen receptors (CARs). The lymphocytes used for infusion can be isolated from a donor (allogeneic ACT), or from the cancer-bearing host (autologous ACT). US2014/109246 discloses methods for the generation of xenochimaeric animals, comprising bone marrow progenitor cells and tumors from a heterologous animal. US2014/255369 relates to the production of red blood cells from hematopoietic stem cells, by differentiating such cells in the presence of a protein that induces cell survival and proliferation US2014/356392 discloses methods of modulation of the viability of a cell, methods of modulating an immune response, and methods of identifying agents capable of modulation of the viability of a cell or an immune response. WO2011/100477 discloses processes for the improved production of antibody producing organisms, antibody producing tissues, antibody producing cells and antibodies.

### SUMMARY OF THE INVENTION

In a first aspect, there is provided a composition for use in adoptive cell therapy for treating a melanoma, wherein the composition comprises:
(a) a MYC fusion peptide, comprising (i) a protein transduction domain, optionally a TAT protein transduction domain sequence; (ii) a MYC polypeptide sequence; and
(b) one or more primary immune cells isolated from a donor subject that has a melanoma tumor, wherein the one or more primary immune cells are reactive against a melanoma-specific antigen.

In the composition for the use of the first aspect of the invention, the MYC fusion peptide may comprise SEQ ID NO: 1. In the composition for the use of the first aspect of the invention the one or more immune cells may comprise one or more lymphocytes, optionally, a T cell, a B cell, an NK cell, or any combination thereof. In the composition for the use of the first aspect of the invention, the one or more lymphocytes may comprise a tumor-infiltrating lymphocyte or may be recombinantly modified to express a modified receptor or chimeric antigen receptor.

In a second aspect of the invention, there is provided a modified immune cell for use in adoptive cell therapy for treating a melanoma in a subject wherein the modified immune cell comprises a MYC fusion peptide comprising (i) a protein transduction domain, optionally a TAT protein transduction domain sequence; and (ii) a MYC polypeptide sequence and is reactive against a tumor-specific antigen. The modified immune cell for the use of the second aspect of the invention may be the MYC fusion peptide comprises SEQ ID NO: 1; and/or the one or more immune cells may comprise one or more lymphocytes, optionally, a T cell, a B cell, an NK cell, or any combination thereof, and optionally the one or more lymphocytes may comprise a tumor-infiltrating lymphocyte or may be recombinantly modified to express a modified receptor or chimeric antigen receptor.

The modified immune cell for the use of the second aspect of the invention may be derived from primary immune cells isolated from the recipient subject or isolated from a separate donor subject having the same type of melanoma. The modified immune cell for the use of the second aspect of the invention may be prepared by contacting the primary immune cells *in vitro* with the MYC fusion peptide following isolation from the donor subject. The modified immune cell for the use of the second aspect of the invention, may further comprise expanding the primary immune cells *in vitro* prior to or following contacting with the MYC fusion peptide, wherein the cells are optionally, expanded using an anti-CD3 antibody, irradiated allogenic feeder cells, or an exogenous cytokine, wherein the cytokine is optionally, interleukin-2. The modified immune cell for the use of the second aspect of the invention, may be for use wherein the recipient subject is:
(i) lymphodepleted prior to administration of the one or more modified immune cells;
(ii) has been administered a cytokine prior to, during, or subsequent to administration of the one or more modified immune cells, wherein the cytokine is optionally selected from a group consisting of interferon α, interferon β, interferon γ, complement C5a, IL-2, TNFalpha, CD40L, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 and XCL2; and/or
(iii) is a human or a non-human animal.

The modified immune cell for the use of the second aspect of the invention may be for use wherein the melanoma is metastatic. The modified immune cell for the use of the second aspect of the invention may be for use wherein the recipient subject has been administered an additional cancer therapy, optionally selected from among chemotherapy, radiation therapy, immunotherapy, monoclonal antibodies, anti-cancer nucleic acids or proteins, anti-cancer viruses or microorganisms, and any combinations thereof.

In third aspect of the invention, there is provided a method for preparing modified immune cells for adoptive cell therapy for melanoma, comprising contacting one or more immune cells in vitro with a MYC fusion polypeptide, wherein the immune cells are from a donor that has been exposed to one or more tumor antigens and wherein the MYC fusion peptide comprises (i) a protein transduction domain, optionally, a TAT protein transduction domain sequence; (ii) a MYC polypeptide sequence and are reactive to a tumor-specific antigen. The one or more modified immune cells may be derived from primary immune cells isolated from a subject having melanoma. The method may comprise expanding the primary immune cells in vitro prior to or following contacting with the MYC fusion peptide; and/or wherein the MYC fusion peptide comprises SEQ ID NO: 1

Provided herein are methods for adoptive cell transfer for the treatment of melanoma. Provided herein are methods for the treatment of melanoma in a subject comprising administering a therapeutically effective amount of immune cells having antitumor activity to the subject, wherein the immune cells are contacted with a protein transduction domain (PTD)-MYC fusion polypeptide prior to administration to the subject. The immune cells may comprise one or more lymphocytes. The one or more lymphocytes may comprise T cells and/or B cells. The one or more lymphocytes may comprise tumor-infiltrating lymphocytes. The melanoma may be a metastatic melanoma. The melanoma may be a superficial spreading melanoma, a nodular melanoma, a lentigo maligna melanoma, or an acral melanoma. The immune cells may be obtained from a donor subject having melanoma. Donor subject and the subject receiving the immune cells may be the same (i.e., autologous ACT). Donor subject and the subject receiving the immune cells may be different (i.e., allogeneic ACT).

The PTD-MYC fusion polypeptide may comprise: (i) an HIV TAT protein transduction domain; and (ii) a MYC polypeptide sequence. The PTD-MYC fusion polypeptide may translocate to the nucleus of the immune cell. The PTD-MYC fusion polypeptide may exhibit a biological activity of MYC, such as the activation of MYC target genes. The fusion peptide may comprise SEQ ID NO: 1.

Described herein, are compositions comprising (a) a MYC fusion peptide, comprising (i) a protein transduction domain; (ii) a MYC polypeptide sequence; and (b) one or more primary immune cells isolated from a donor subject that has a melanoma tumor, wherein the one or more primary immune cells are reactive against a melanoma-specific antigen. The MYC fusion peptide may translocate to the nucleus of the one or more primary immune cells. The MYC fusion peptide may exhibit a biological activity of MYC. The MYC fusion peptide may further comprises one or more molecules that link the protein transduction domain and the MYC polypeptide. The MYC fusion peptide may comprise a MYC fusion peptide with the following general structure:
protein transduction domain-X-MYC sequence,
wherein -X- is molecule that links the protein transduction domain and the MYC sequence. The protein transduction domain sequence may be a TAT protein transduction domain sequence. The TAT protein transduction domain sequence may be selected from the group consisting of TAT[48-57] and TAT[57-48]. The MYC fusion peptide may comprise SEQ ID NO: 1. The MYC fusion peptide may be acetylated. The one or more immune cells may have antitumor activity against melanoma cells. The one or more immune cells may comprise one or more lymphocytes. The one or more lymphocytes may comprise a T cell, a B cell, an NK cell, or any combination thereof. The T cell may be selected from the group consisting of naive T cells, CD4+ T cells, CD8+ T cells, memory T cells, activated T cells, anergic T cells, tolerant T cells, chimeric T cells, and antigen-specific T cells. The B cells may be selected from the group consisting of naive B cells, plasma B cells, activated B cells, memory B cells, anergic B cells, tolerant B cells, chimeric B cells, and antigen-specific B cells. The one or more lymphocytes may be a tumor-infiltrating lymphocyte, T-cell receptor modified lymphocyte, or a chimeric antigen receptor modified lymphocyte. The tumor-infiltrating lymphocyte may have a CD8+CD25+ signature. The tumor-infiltrating lymphocyte may have a CD4+CD25+ signature. The one or more immune cells may comprise a detectable moiety.

Described herein are methods for treating a melanoma in a subject, comprising administering one or more modified immune cells to the subject in need thereof, wherein the one or more modified immune cells comprise a MYC fusion peptide comprising (i) a protein transduction domain; (ii) a MYC polypeptide sequence and are reactive to a tumor-specific antigen. The one or more modified immune cells may be derived from primary immune cells isolated from the subject. The one or more modified immune cells may be derived from primary immune cells isolated from a separate donor subject having the same type of melanoma. The one or more modified immune cells may be prepared by contacting the primary immune cells in vitro with the MYC fusion peptide following isolation. The methods may further comprise expanding the primary immune cells *in vitro* prior to contacting with the MYC fusion peptide. The methods may further comprise expanding the primary immune cells following contacting with the MYC fusion peptide. The cells may be expanded using an anti-CD3 antibody. The cells may be expanded using an irradiated allogenic feeder cells. The cells may be expanded in the presence of an exogenous cytokine. The cytokine may be interleukin-2. The MYC fusion peptide translocates to the nucleus of the immune cell. The MYC fusion peptide may exhibit a biological activity of MYC. The MYC fusion peptide may further comprise one or more molecules that link the protein transduction domain and the MYC polypeptide. The MYC fusion peptide may comprise a MYC fusion peptide with the following general structure:
protein transduction domain-X-MYC sequence,
wherein -X- is molecule that links the protein transduction domain and the MYC sequence. The protein transduction domain sequence may be a TAT protein transduction domain sequence. The TAT protein transduction domain sequence may be selected from the group consisting of TAT[48-57] and TAT[57-48]. The MYC fusion peptide may comprise SEQ ID NO: 1. The MYC fusion peptide may be acetylated. The one or more modified immune cells may have antitumor activity against melanoma cells in the subject. The one or more modified immune cells may have antitumor activity against melanoma cells in the subject. The one or more modified immune cells may comprise one or more anergic immune cells. The one or more immune cells may comprise one or more lymphocytes. The one or more lymphocytes may comprise a T cell, a B cell, an NK, or any combination thereof. The T cell may be selected from the group consisting of naive T cells, CD4+ T cells, CD8+ T cells, memory T cells, activated T cells, anergic T cells, tolerant T cells, chimeric T cells, and antigen-specific T cells. The B cells may be selected from the group consisting of naive B cells, plasma B cells, activated B cells, memory B cells, anergic B cells, tolerant B cells, chimeric B cells, and antigen-specific B cells. The one or more lymphocytes may be a tumor-infiltrating lymphocyte, T-cell receptor modified lymphocyte, or a chimeric antigen receptor modified lymphocyte. The lymphocyte may have a CD8+CD28-CD152- signature. The lymphocyte may have a CD8+CD25+ signature. The lymphocyte may have a CD4+CD25+ signature. The methods may further comprise isolating the primary immune cells from the donor subject. The donor subject may have melanoma. The one or more modified immune cells may be administered intravenously, intraperitoneally, subcutaneously, intramuscularly, or intratumorally. The methods may further comprise lymphodepleting the subject prior to administration of the one or more modified immune cells. The methods may further comprise administering a cytokine to the subject. The cytokine may be administered prior to, during, or subsequent to administration of the one or more modified immune cells. The cytokine may be selected from a group consisting of interferon α, interferon β, interferon γ, complement C5a, IL-2, TNFalpha, CD40L, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 and XCL2. The melanoma may be metastatic. The subject may be a human or an animal. The methods may further comprise administering an additional cancer therapy. The additional cancer therapy may be selected from among chemotherapy, radiation therapy, immunotherapy, monoclonal antibodies, anti-cancer nucleic acids or proteins, anti-cancer viruses or microorganisms, and any combinations thereof. Tthe one or more modified immune cells may comprise a detectable moiety.

Also described herein, in certain embodiments are methods for preparing modified immune cells for melanoma therapy, comprising contacting one or more immune cells in vitro with a MYC fusion polypeptide, wherein the immune cells are from a donor that has been exposed to one or more tumor antigens and wherein the MYC fusion peptide comprises (i) a protein transduction domain; (ii) a MYC polypeptide sequence and are reactive to a tumor-specific antigen. In some embodiments, the one or more modified immune cells are derived from primary immune cells isolated from a subject having melanoma. In some embodiments, the methods further comprise expanding the primary immune cells in vitro prior to contacting with the MYC fusion peptide. In some embodiments, the methods further comprise expanding the primary immune cells following contacting with the MYC fusion peptide. The cells may be expanded using an anti-CD3 antibody. The cells may be expanded using an irradiated allogenic feeder cells. The cells may be expanded in the presence of an exogenous cytokine. In some embodiments, the cytokine is interleukin-2. The MYC fusion peptide may translocate to the nucleus of the immune cell. The MYC fusion peptide may exhibit a biological activity of MYC. The MYC fusion peptide may further comprise one or more molecules that link the protein transduction domain and the MYC polypeptide. The MYC fusion peptide may comprise a MYC fusion peptide with the following general structure:
protein transduction domain-X-MYC sequence,
wherein -X- is molecule that links the protein transduction domain and the MYC sequence. The protein transduction domain sequence may be a TAT protein transduction domain sequence. The TAT protein transduction domain sequence may be selected from the group consisting of TAT[48-57] and TAT[57-48]. In some embodiments, the MYC fusion peptide comprises SEQ ID NO: 1. The MYC fusion peptide may be acetylated. The one or more modified immune cells may have antitumor activity. The one or more modified immune cells may have antitumor activity against melanoma cells in the subject. The one or more modified immune cells may comprise one or more anergic immune cells. The one or more immune cells may comprise one or more lymphocytes. The one or more lymphocytes may comprise a T cell, a B cell, an NK, or any combination thereof. The T cell may selected from the group consisting of naive T cells, CD4+ T cells, CD8+ T cells, memory T cells, activated T cells, anergic T cells, tolerant T cells, chimeric T cells, and antigen-specific T cells. The B cells may be selected from the group consisting of naive B cells, plasma B cells, activated B cells, memory B cells, anergic B cells, tolerant B cells, chimeric B cells, and antigen-specific B cells. The one or more lymphocytes may be a tumor-infiltrating lymphocyte, T-cell receptor modified lymphocyte, or a chimeric antigen receptor modified lymphocyte. The lymphocyte may have a CD8+CD28-CD152- signature. The lymphocyte may have a CD8+CD25+ signature. The lymphocyte may have a CD4+CD25+ signature.

Also described herein, are compositions comprising: (a) one or more isolated primary immune cells that have been exposed to a melanoma cell line; and (b) a MYC fusion peptide, comprising (i) a protein transduction domain; (ii) a MYC polypeptide sequence; wherein the one or more primary immune cells are reactive against a melanoma-specific antigen.

Also described herein, in certain embodiments, are any of the aforementioned compositions for use in treating a melanoma. Also described herein, are any of the aforementioned compositions for use in the manufacture of a medicament for use in treating a melanoma.

Also described herein, are methods for increasing the efficacy of adoptive cell therapy or T-cell therapy in a subject comprising administering any of the aforementioned compositions.

Also described herein, are modified tumor-infiltrating lymphocytes comprising a MYC fusion peptide, comprising (i) a protein transduction domain; (ii) a MYC polypeptide sequence. The tumor-infiltrating lymphocytes may be derived from primary tumor-infiltrating lymphocytes isolated from a subject that has cancer (e.g., melanoma).

Also described herein, are lymphocytes comprising a chimeric antigen receptor and a MYC fusion peptide, comprising (i) a protein transduction domain; (ii) a MYC polypeptide sequence. The lymphocytes are derived from primary lymphocytes isolated from a subject that has cancer (e.g., melanoma).

Also described herein, are methods for preparing a composition for adoptive cell therapy comprising contacting one or more primary immune cells with MYC fusion peptide, comprising (i) a protein transduction domain; (ii) a MYC polypeptide sequence, wherein one or more primary immune cells are isolated from a patient having melanoma, and wherein one or more primary immune cells are reactive to a melanoma-specific antigen.

Also provided are kits comprising the MYC-fusion polypeptides and/or MYC-fusion polypeptide-modified immune cells provided herein for use in treating a melanoma. The kit may comprise one for more reagents for the detection of the administered MYC-fusion polypeptides and/or MYC-fusion polypeptide-modified immune cells. The kit may comprise cells for treatment with a MYC-fusion polypeptide provided herein, for example, hematopoietic stem cells, donor leukocytes, T cells, or NK cells. The kit may comprise associated instructions for using the MYC-fusion polypeptides and/or MYC-fusion polypeptide-modified immune cells.

### BRIEF DESCRIPTION OF THE DRAWINGS

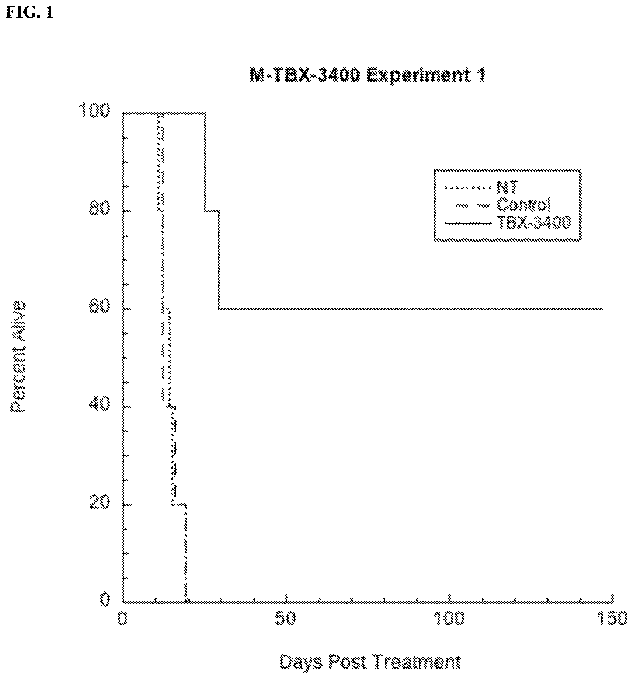

**FIG. 1** illustrates results for survival of melanoma tumor-bearing mice following infusion of lymphocytes from tumor -bearing donor mice treated with TAT-MYC for 1 hour. Mice were treated with TAT-MYC lymphocytes, lymph cells treated with a control protein or left untreated. Day of death recorded with day of treatment as Day 0.

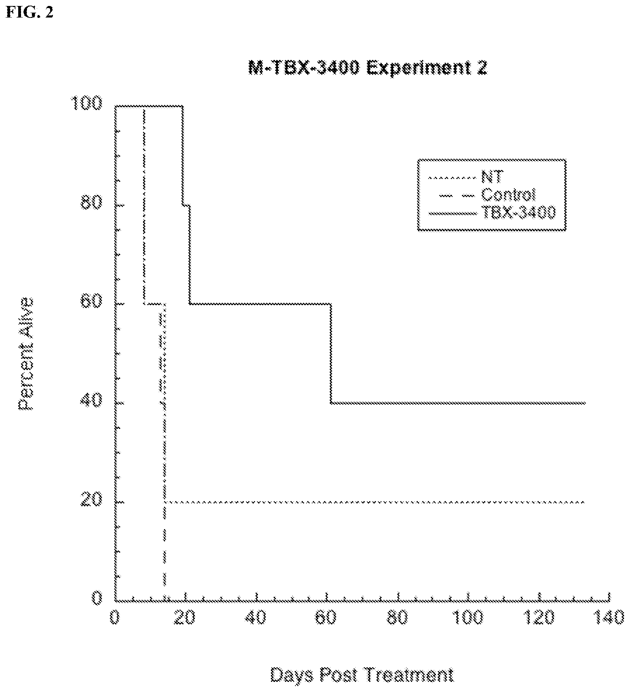

**FIG. 2** illustrates results for survival of melanoma tumor-bearing mice following infusion of lymphocytes from tumor -bearing donor mice treated with TAT-MYC (repeat of experiment shown in Fig. 1). Mice were treated with TAT-MYC lymphocytes, lymph cells treated with a control protein or left untreated. Day of death recorded with day of treatment as Day 0.

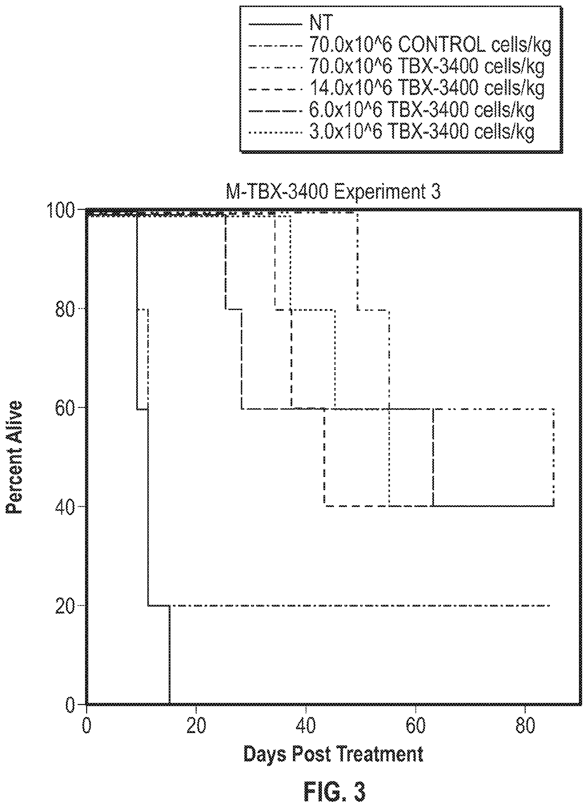

**FIG. 3** illustrates results for survival of melanoma tumor-bearing mice following infusion of different amounts of lymphocytes from tumor-bearing donor mice treated with TAT-MYC. Mice were treated with TAT-MYC lymphocytes, lymph cells treated with a control protein or left untreated. Day of death recorded with day of treatment as Day 0.

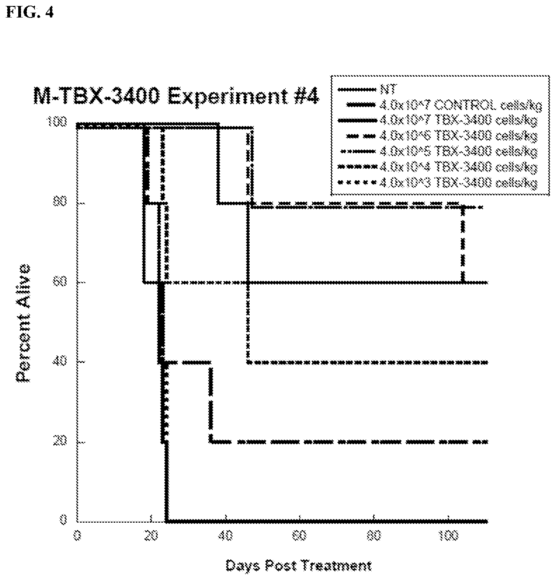

**FIG. 4** illustrates results for survival of melanoma tumor-bearing mice following infusion of different amounts of lymphocytes from tumor-bearing donor mice treated with TAT-MYC. Mice were treated with TAT-MYC lymphocytes, lymph cells treated with a control protein or left untreated. Day of death recorded with day of treatment as Day 0.

### DETAILED DESCRIPTION OF THE INVENTION

The present disclosure is not to be limited in terms of the particular embodiments described in this application, which are intended as single illustrations of individual aspects of the disclosure. All the various embodiments of the present disclosure will not be described herein. Many modifications and variations of the disclosure can be made without departing from its scope, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled.

It is to be understood that the present disclosure is not limited to particular uses, methods, reagents, compounds, compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

Unless defined otherwise, all technical and scientific terms used herein have the same meaning as commonly understood by one of ordinary skill in the art to which this disclosure belongs.

### I. Definitions

The terminology used herein is for the purpose of describing particular embodiments only and is not intended to be limiting of the disclosure. As used herein, the singular forms "a", "an" and "the" are intended to include the plural forms as well, unless the context clearly indicates otherwise.

As used herein, the term "about" means that a value can vary +/- 20%, +/- 15%, +/- 10% or +/- 5% and remain within the scope of the present disclosure. For example, "a concentration of about 200 IU/mL" encompasses a concentration between 160 IU/mL and 240 IU/mL.

As used herein, the term "administration" of an agent to a subject includes any route of introducing or delivering the agent to a subject to perform its intended function. Administration can be carried out by any suitable route, including intravenously, intramuscularly, intraperitoneally, or subcutaneously. Administration includes self-administration and the administration by another.

The term "amino acid" refers to naturally occurring and non-naturally occurring amino acids, as well as amino acid analogs and amino acid mimetics that function in a manner similar to the naturally occurring amino acids. Naturally encoded amino acids are the 20 common amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine) and pyrolysine and selenocysteine. Amino acid analogs refers to agents that have the same basic chemical structure as a naturally occurring amino acid, *i.e.,* an α carbon that is bound to a hydrogen, a carboxyl group, an amino group, and an R group, such as, homoserine, norleucine, methionine sulfoxide, methionine methyl sulfonium. Such analogs have modified R groups (such as, norleucine) or modified peptide backbones, but retain the same basic chemical structure as a naturally occurring amino acid. Amino acids forming a polypeptide may be in the D form. The amino acids forming a polypeptide may be in the L form. A first plurality of amino acids forming a polypeptide may be in the D form and a second plurality are in the L form.

Amino acids are referred to herein by either their commonly known three letter symbols or by the one-letter symbols recommended by the IUPAC-IUB Biochemical Nomenclature Commission. Nucleotides, likewise, are referred to by their commonly accepted single-letter code.

The terms "polypeptide," "peptide," and "protein" are used interchangeably herein to refer to a polymer of amino acid residues. The terms apply to naturally occurring amino acid polymers as well as amino acid polymers in which one or more amino acid residues is a non-naturally occurring amino acid, *e.g.,* an amino acid analog. The terms encompass amino acid chains of any length, including full length proteins, wherein the amino acid residues are linked by covalent peptide bonds.

As used herein, a "control" is an alternative sample used in an experiment for comparison purpose. A control can be "positive" or "negative." For example, where the purpose of the experiment is to determine a correlation of the efficacy of a therapeutic agent for the treatment for a particular type of disease, a positive control (a composition known to exhibit the desired therapeutic effect) and a negative control (a subject or a sample that does not receive the therapy or receives a placebo) are typically employed.

As used herein, the term "effective amount" or "therapeutically effective amount" refers to a quantity of an agent sufficient to achieve a desired therapeutic effect. In the context of therapeutic applications, the amount of a therapeutic peptide administered to the subject can depend on the type and severity of the infection and on the characteristics of the individual, such as general health, age, sex, body weight and tolerance to drugs. It can also depend on the degree, severity and type of disease. The skilled artisan will be able to determine appropriate dosages depending on these and other factors.

As used herein, the term "expression" refers to the process by which polynucleotides are transcribed into mRNA and/or the process by which the transcribed mRNA is subsequently being translated into peptides, polypeptides, or proteins. If the polynucleotide is derived from genomic DNA, expression can include splicing of the mRNA in a eukaryotic cell. The expression level of a gene can be determined by measuring the amount of mRNA or protein in a cell or tissue sample. The expression level of a gene from one sample can be directly compared to the expression level of that gene from a control or reference sample. The expression level of a gene from one sample can be directly compared to the expression level of that gene from the same sample following administration of the compositions disclosed herein. The term "expression" also refers to one or more of the following events: (1) production of an RNA template from a DNA sequence (*e.g*., by transcription) within a cell; (2) processing of an RNA transcript (*e.g.*, by splicing, editing, 5' cap formation, and/or 3' end formation) within a cell; (3) translation of an RNA sequence into a polypeptide or protein within a cell; (4) post-translational modification of a polypeptide or protein within a cell; (5) presentation of a polypeptide or protein on the cell surface; and (6) secretion or presentation or release of a polypeptide or protein from a cell.

The term "linker" refers to synthetic sequences (*e.g*., amino acid sequences) that connect or link two sequences, *e.g*., that link two polypeptide domains. The linker may contain 1, 2, 3, 4, 5, 6, 7, 8, 9, or 10 of amino acid sequences.

The terms "lyophilized," "lyophilization" and the like as used herein refer to a process by which the material (*e.g*., nanoparticles) to be dried is first frozen and then the ice or frozen solvent is removed by sublimation in a vacuum environment. An excipient can be included in pre-lyophilized formulations to enhance stability of the lyophilized product upon storage. The lyophilized sample can further contain additional excipients.

As used herein the term immune cell refers to any cell that plays a role in the immune response. Immune cells are of hematopoietic origin, and include lymphocytes, such as B cells and T cells; natural killer cells; myeloid cells, such as monocytes, macrophages, dendritic cells, eosinophils, neutrophils, mast cells, basophils, and granulocytes.

The term "lymphocyte" refers to all immature, mature, undifferentiated and differentiated white lymphocyte populations including tissue specific and specialized varieties. It encompasses, by way of non-limiting example, B cells, T cells, NKT cells, and NK cells. Lymphocytes may include all B cell lineages including pre-B cells, progenitor B cells, early pro-B cells, late pro-B cells, large pre-B cells, small pre-B cells, immature B cells, mature B cells, plasma B cells, memory B cells, B-1 cells, B-2 cells and anergic AN1/T3 cell populations.

As used herein, the term T-cell includes naive T cells, CD4+ T cells, CD8+ T cells, memory T cells, activated T cells, anergic T cells, tolerant T cells, chimeric T cells, and antigen-specific T cells.

The term "B cell" or "B cells" refers to, by way of non-limiting example, a pre-B cell, progenitor B cell, early pro-B cell, late pro-B cell, large pre-B cell, small pre-B cell, immature B cell, mature B cell, naive B cells, plasma B cells, activated B cells, anergic B cells, tolerant B cells, chimeric B cells, antigen-specific B cells, memory B cell, B-1 cell, B-2 cells and anergic AN1/T3 cell populations. The term B cell may include a B cell that expresses an immunoglobulin heavy chain and/or light chain on its cells surface. The term B cell may include a B cell that expresses and secretes an immunoglobulin heavy chain and/or light chain. The term B cell may include a cell that binds an antigen on its cell-surface. B cells or AN1/T3 cells may be utilized in the processes described. Such cells may be optionally substituted with any animal cell suitable for expressing, capable of expressing (*e.g*., inducible expression), or capable of being differentiated into a cell suitable for expressing an antibody including, *e.g.,* a hematopoietic stem cell, a naive B cell, a B cell, a pre-B cell, a progenitor B cell, an early Pro-B cell, a late pro-B cell, a large pre-B cell, a small pre-B cell, an immature B cell, a mature B cell, a plasma B cell, a memory B cell, a B-1 cell, a B-2 cell, an anergic B cell, or an anergic AN1/T3 cell.

As used herein "adoptive cell therapeutic composition" refers to any composition comprising cells suitable for adoptive cell transfer. The adoptive cell therapeutic composition may comprise a cell type selected from a group consisting of a tumor infiltrating lymphocyte (TIL), TCR (i.e. heterologous T-cell receptor) modified lymphocytes and CAR (i.e. chimeric antigen receptor) modified lymphocytes. The adoptive cell therapeutic composition may comprise a cell type selected from a group consisting of T-cells, CD8+ cells, CD4+ cells, NK-cells, delta-gamma T-cells, regulatory T-cells and peripheral blood mononuclear cells. TILs, T-cells, CD8+ cells, CD4+ cells, NK-cells, delta-gamma T-cells, regulatory T-cells or peripheral blood mononuclear cells may form the adoptive cell therapeutic composition. he adoptive cell therapeutic composition may comprise T cells.

As used herein "tumor-infiltrating lymphocytes" or TILs refer to white blood cells that have left the bloodstream and migrated into a tumor.

The terms "MYC" and "MYC gene" are synonyms. They refer to a nucleic acid sequence that encodes a MYC polypeptide. A MYC gene comprises a nucleotide sequence of at least 120 nucleotides that is at least 60% to 100% identical or homologous, *e.g*., at least 60, 65%, 70%, 75%, 80%, 85%, 86%, 87%, 88%, 90%, 91%, 92%, 94%, 95%, 96%, 97%, 98%, or any other percent from about 70% to about 100% identical to sequences of NCBI Accession Number NM-002467. The MYC gene may be a proto-oncogene. In certain instances, a MYC gene is found on chromosome 8, at 8q24.21. In certain instances, a MYC gene begins at 128,816,862 bp from pter and ends at 128,822,856 bp from pter. In certain instances, a MYC gene is about 6 kb. In certain instances, a MYC gene encodes at least eight separate mRNA sequences-5 alternatively spliced variants and 3 unspliced variants.

The terms "MYC protein," "MYC polypeptide," and "MYC sequence" are synonyms and refer to the polymer of amino acid residues disclosed in NCBI Accession Number UniProtKB/Swiss-Prot:P01106.1 (MYC isoform 1) or NP_002458.2 (UniProtKB/Swiss-Prot:P01106.2; MYC isoform 2), and functional homologs, analogs or fragments thereof. The sequence of or UniProtKB/Swiss-Prot:P01106.1 is: The sequence of NP_002458.2 (UniProtKB/Swiss-Prot:P01106.2) is:

The MYC polypeptide may be a complete MYC polypeptide sequence. The MYC polypeptide may be a partial MYC polypeptide sequence. The MYC polypeptide may comprise at least 400 consecutive amino acids of SEQ ID NO: 2 OR 11. The MYC polypeptide may comprise at least 400 consecutive amino acids of SEQ ID NO: 2 OR 11 and retains at least one MYC activity. The MYC polypeptide may comprise at least 400, at least 410, at least 420, at least 430, or at least 450 consecutive amino acids of SEQ ID NO: 2 OR 11. The MYC polypeptide may comprise at least 400, at least 410, at least 420, at least 430, or at least 450 consecutive amino acids of SEQ ID NO: 2 OR 11 and retains at least one MYC activity. The MYC polypeptide may be c-MYC. The MYC polypeptide sequence may comprise the sequence shown below:

The MYC polypeptide sequence comprises the sequence shown below:

A MYC polypeptide may comprise an amino acid sequence that is at least 40% to 100% identical, *e.g.,* at least 40%, 45%, 50%, 55%, 60%, 65%, 70%, 75%, 80%, 81%, 82%, 83%, 84%, 85%, 86%, 87%, 88%, 90%, 91%, 92%, 94%, 95%, 96%, 97%, 98%, 99%, or any other percent from about 40% to about 100% identical to the sequence of NCBI Accession Number NP002458.2 or UniProtKB/Swiss-Prot Accession Number P01106.1. MYC polypeptide may refer to a polymer of 439 amino acids, a MYC polypeptide that has not undergone any post-translational modifications. MYC polypeptide may refer to a polymer of 439 amino acids that has undergone post-translational modifications. The MYC polypeptide may be 48,804 kDa. The MYC polypeptide may contain a basic Helix-Loop-Helix Leucine Zipper (bHLH/LZ) domain. The bHLH/LZ domain may comprise the sequence of: ELKRSFFALRDQIPELENNEKAPKVVILKKATAYILSVQAEEQKLISEEDLLRKRREQL KHKLEQLR (SEQ ID NO: 5). The MYC polypeptide may be a transcription factor (*e.g.,* Transcription Factor 64). The MYC polypeptide may contain an E-box DNA binding domain. The MYC polypeptide may bind to a sequence comprising CACGTG. The MYC polypeptide may promote one or more of cell survival and/or proliferation. A MYC polypeptide may include one or more of those described above, and include one or more post-translational modifications (*e.g*., acetylation). The MYC polypeptides may comprise one or more additional amino acid residues at the N-terminus or C-terminus of the polypeptide. The MYC polypeptides may be fusion proteins. The MYC polypeptides may be linked to one or more additional peptides at the N-terminus or C-terminus of the polypeptide.

Proteins suitable for use in the methods described herein also includes functional variants, including proteins having between 1 to 15 amino acid changes, *e.g*., 1, 2, 3, 4, 5, 6, 7, 8, 9, 10, 11, 12, 13, 14, or 15 amino acid substitutions, deletions, or additions, compared to the amino acid sequence of any protein described herein. The altered amino acid sequence may be at least 75% identical, *e.g.,* 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identical to the amino acid sequence of any protein inhibitor described herein. Such sequence-variant proteins are suitable for the methods described herein as long as the altered amino acid sequence retains sufficient biological activity to be functional in the compositions and methods described herein. Where amino acid substitutions are made, the substitutions can be conservative amino acid substitutions. Among the common, naturally occurring amino acids, for example, a "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine. The BLOSUM62 table is an amino acid substitution matrix derived from about 2,000 local multiple alignments of protein sequence segments, representing highly conserved regions of more than 500 groups of related proteins (Henikoff et al., (1992), Proc. Natl Acad. Sci. USA, 89:10915- 10919). Accordingly, the BLOSUM62 substitution frequencies are used to define conservative amino acid substitutions that, may be introduced into the amino acid sequences described or disclosed herein. Although it is possible to design amino acid substitutions based solely upon chemical properties (as discussed above), the language "conservative amino acid substitution" preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (*e.g.,* 1, 2 or 3), while more preferred conservative amino acid substitutions are characterized by a BLOSUM62 value of at least 2 (*e.g.,* 2 or 3).

The phrases "E-box sequence" and "enhancer box sequence" are used interchangeably herein and mean the nucleotide sequence CANNTG, wherein N is any nucleotide. In certain instances, the E-box sequence comprises CACGTG. In certain instances, the basic helix-loop-helix domain of a transcription factor encoded by MYC binds to the E-box sequence. In certain instances the E-box sequence is located upstream of a gene *(e.g.,* p21, Bc1-2, or ornithine decarboxylase). In certain instances, the MYC polypeptide contains an E-box DNA binding domain. In certain instances, the E-box DNA binding domain comprises the sequence of KRRTHNVLERQRRN (SEQ ID NO: 6). In certain instances, the binding of the transcription factor encoded by MYC to the E-box sequence, allows RNA polymerase to transcribe the gene downstream of the E-box sequence.

The term "MYC activity" or "MYC biological activity" or "biologically active MYC" includes one or more of enhancing or inducing cell survival, cell proliferation, and/or antibody production. By way of example and not by way of limitation, MYC activity includes enhancement of expansion of anti-CD3 and anti-CD28 activated T-cells and/or increased proliferation of long-term self-renewing hematopoietic stem cells. MYC activity also includes entry into the nucleus of a cell, binding to a nucleic acid sequence (*e.g*., binding an E-box sequence), and/or inducing expression of MYC target genes.

The terms "patient," "subject," "individual," and the like are used interchangeably herein, and refer to an animal, typically a mammal. The patient, subject, or individual may be a mammal. The patient, subject or individual may be a human. The patient, subject or individual may be an animal, such as, but not limited to, domesticated animals, such as equine, bovine, murine, ovine, canine, and feline.

The terms "protein transduction domain (PTD)" or "transporter peptide sequence" (also known as cell permeable proteins (CPP) or membrane translocating sequences (MTS)) are used interchangeably herein to refer to small peptides that are able to ferry much larger molecules into cells independent of classical endocytosis. A nuclear localization signal can be found within the protein transduction domain, which mediates further translocation of the molecules into the cell nucleus.

The terms "treating" or "treatment" as used herein covers the treatment of a disease in a subject, such as a human, and includes: (i) inhibiting a disease, *i.e.,* arresting its development; (ii) relieving a disease, *i.e.,* causing regression of the disease; (iii) slowing progression of the disease; and/or (iv) inhibiting, relieving, or slowing progression of one or more symptoms of the disease. With respect to a melanoma, "treating" or "treatment" also encompasses regression of a tumor, slowing tumor growth, inhibiting metastasis of a melanoma tumor, inhibiting relapse or recurrent melanoma and/or maintaining remission.

It is also to be appreciated that the various modes of treatment or prevention of medical diseases and conditions as described are intended to mean "substantial," which includes total but also less than total treatment or prevention, and wherein some biologically or medically relevant result is achieved. The treatment can be a continuous prolonged treatment for a chronic disease or a single, or few time administrations for the treatment of an acute condition.

The term "therapeutic" as used herein means a treatment and/or prophylaxis. A therapeutic effect is obtained by suppression, remission, or eradication of a disease state.

### II. Overview

The present disclosure relates, in part, to the treatment of melanoma in a subject by administering a composition comprising one or more immune cells having anti-tumor activity (e.g., immune cells that modulate a response against a tumor, such as tumor-infiltrating lymphocytes (TILs)), wherein the one or more immune cells are contacted with a PTD-MYC fusion polypeptide *in vitro* prior to administration to the subject. The immune cells may be obtained from a donor subject that has a melanoma tumor. The cells may be autologous to the subject receiving treatment. The melanoma may be a superficial spreading melanoma, a nodular melanoma, a lentigo maligna melanoma, or an acral melanoma.

The present disclosure is based, at least in part, on the discovery, that treating lymphocytes isolated from a donor subject having a melanoma tumor with a MYC fusion polypeptide containing a MYC polypeptide and a protein transduction domain (PTD), such as the HIV TAT protein transduction domain, and administering the treated lymphocytes to a subject bearing a melanoma tumor significantly increases the survival of the tumor-bearing subject. The examples provided herein demonstrate that immune cells extracted from the lymph nodes of a melanoma-bearing mouse had significantly increased therapeutic efficacy when the cells were treated with a TAT-MYC fusion protein *in vitro* prior to administration to a second melanoma-bearing mice. These data support that adoptive cell transfer using anti-tumor immune cells treated with a PTD-MYC fusion polypeptide can be employed in the treatment of tumors, such as melanoma tumors.

The method for the treatment of melanoma in a subject may comprise administering immune cells that have been contacted *in vitro* with a PTD-MYC fusion polypeptide. The immune cells for use in the present methods may be primed *in vivo* with melanoma tumor antigen. The immune cells maybe from a donor having melanoma. The immune cells may be from a donor having a solid tumor, such as a melanoma tumor. The immune cells may be contacted *in vivo* with a melanoma tumor antigen. The immune cells may be from a donor that has been exposed to a one or more melanoma tumor antigens. The immune cells may be from a donor that has been exposed to an anti-tumor vaccine. The immune cells may be B cells, T cells, NK cells, or any combination thereof. The immune cells may be tumor infiltrating lymphocytes (TIL). The immune cells may be chimeric antigen receptor (CAR)-T cells.

The method for the treatment of melanoma in a subject may comprise administering one or more modified immune cells to the subject in need thereof, wherein the one or more modified immune cells comprise a MYC fusion peptide comprising (i) a protein transduction domain; (ii) a MYC polypeptide sequence and are reactive to a melanoma tumor-specific antigen.

The method for the treatment of melanoma in a subject may comprise the steps of:
a) contacting immune cells *in vitro* with a MYC fusion polypeptide, wherein the immune cells are from a donor that has been exposed to one or more melanoma tumor antigens and the MYC fusion peptide comprising (i) a protein transduction domain; (ii) a MYC polypeptide sequence; and
b) administering the contacted immune cells to the melanoma tumor-bearing subject, whereby the melanoma is treated.

Contacting the immune cells *in vitro* with a PTD-MYC fusion polypeptide may be performed by culturing the immune cells in the presence of the MYC fusion polypeptide. The immune cells may be cultured in the presence of one or more cytokines and/or growth factors (e.g., interleukin-2 (IL-2), IL-4, IL-7, IL-9, and IL-15). The immune cells may not be expanded prior to administration. The immune cells may be expanded prior to administration. The donor and subject for treatment may be the same.

The immune cells may be tumor-infiltrating lymphocytes. The tumor-infiltrating lymphocytes may be autologous tumor-infiltrating lymphocytes. The method for the treatment of melanoma in a subject may comprise administering lymphocytes that have been contacted *in vitro* with a PTD-MYC fusion polypeptide, wherein the immune cells are from lymphocytes are autologous tumor-infiltrating lymphocytes from the subject.

The method for the treatment of melanoma in a subject may comprise the steps of:
a) contacting lymphocytes *in vitro* with a PTD-MYC fusion polypeptide, wherein the lymphocytes are autologous tumor-infiltrating lymphocytes from the subject, and
b) administering the contacted autologous tumor-infiltrating lymphocytes to the subject, whereby the melanoma is treated.

### Methods of Obtaining and Preparing Immune Cells For Transfer

Immune cells for use in the methods provided herein can be obtained using any suitable method known in the art. In some embodiments, the immune cells are primary immune cells. In some embodiments, the immune cells are lymphocytes, such as T and B cells. In some embodiments, the immune cells are natural killer (NK) cells. In some embodiments, the immune cells are a mixture of lymphocytes and NK cells. The immune cells may be peripheral blood mononuclear cells (PBMC). In some embodiments, the immune cells are T cells that have infiltrated a tumor (e.g., tumor infiltrating lymphocytes). The T cells may be removed during surgery of a melanoma tumor or a metastatic tumor in a subject. For example, the T cells may be isolated after removal of tumor tissue by biopsy. The immune cells may be modified following isolation from a donor. The immune cells may be chimeric antigen receptor (CAR)-T cells.

The T cells may be isolated from sample containing a population of cells, such as a blood, lymph or tissue biopsy sample. T cells can be isolated from a population of cells by any means known in the art. The method may comprise obtaining a bulk population of T cells from a tumor sample by any suitable method known in the art. For example, a bulk population of T cells can be obtained from a tumor sample by dissociating the tumor sample into a cell suspension from which specific cell populations can be selected. Suitable methods of obtaining a bulk population of T cells can include, but are not limited to, any one or more of mechanically dissociating (e.g., mincing) the tumor, enzymatically dissociating (e.g., digesting) the tumor, and aspiration (e.g., as with a needle).

The bulk population of T cells obtained from a tumor sample can comprise any suitable type of T cell. Preferably, the bulk population of T cells obtained from a tumor sample comprises tumor infiltrating lymphocytes (TILs).

The tumor sample can be obtained from any mammal. Unless stated otherwise, as used herein, the term "mammal" refers to any mammal including, but not limited to, mammals of the order Logomorpha, such as rabbits; the order Carnivora, including Felines (cats) and Canines (dogs); the order Artiodactyla, including Bovines (cows) and Swines (pigs); or of the order Perssodactyla, including Equines (horses). The mammals can be non-human primates, e.g., of the order Primates, Ceboids, or Simoids (monkeys) or of the order Anthropoids (humans and apes). The mammal can be a mammal of the order Rodentia, such as mice and hamsters. Preferably, the mammal is a non-human primate or a human. An exemplary mammal is a human. The subject to receive the immune cells may also be the donor of the tumor sample (i.e., autologous ACT)

T cells can be obtained from a number of sources, including peripheral blood mononuclear cells, bone marrow, lymph node tissue, spleen tissue, and tumors. The T cells can be obtained from a unit of blood collected from a subject using any number of techniques known to the skilled artisan, such as Ficoll separation. Cells from the circulating blood of an individual may be obtained by apheresis or leukopheresis. The apheresis product typically contains lymphocytes, including T cells, monocytes, granulocytes, B cells, other nucleated white blood cells, red blood cells, and platelets. The cells collected by apheresis can be washed to remove the plasma fraction and to place the cells in an appropriate buffer or media for subsequent processing steps. The cells may be washed with phosphate buffered saline (PBS). The wash solution may lack calcium and can lack magnesium or can lack many if not all divalent cations. Initial activation steps in the absence of calcium lead to magnified activation. As those of ordinary skill in the art would readily appreciate, a washing step can be accomplished by methods known to those in the art, such as by using a semi-automated "flow-through" centrifuge (for example, the Cobe 2991 cell processor) according to the manufacturer's instructions. After washing, the cells can be resuspended in a variety of biocompatible buffers, such as, for example, Ca-free, Mg-free PBS. Alternatively, the undesirable components of the apheresis sample can be removed and the cells directly resuspended in culture media.

T cells may be isolated from peripheral blood lymphocytes by lysing the red blood cells and depleting the monocytes, for example, by centrifugation through a PERCOLL^{™} gradient. A specific subpopulation of T cells, such as CD28+, CD4+, CDC, CD45RA+, and CD45RO+ T cells, can be further isolated by positive or negative selection techniques. T cells may be isolated by incubation with anti-CD3/anti-CD28 (i.e., 3×28)-conjugated beads, such as DYNABEADS^{®} M-450 CD3/CD28 T, or XCYTE DYNABEADS^{™} for a time period sufficient for positive selection of the desired T cells. The time period may be about 30 minutes. The time period may range from 30 minutes to 36 hours or longer and all integer values there between. The time period may be at least 1, 2, 3, 4, 5, or 6 hours. The time period may be 10 to 24 hours. The incubation time period may be 24 hours. For isolation of T cells from patients with leukemia, use of longer incubation times, such as 24 hours, can increase cell yield. Longer incubation times can be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells.

Enrichment of a T cell population by negative selection can be accomplished with a combination of antibodies directed to surface markers unique to the negatively selected cells. The method may be cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD11b, CD16, HLA-DR, and CD8.

Further, monocyte populations (i.e., CD14+ cells) can be depleted from blood preparations by a variety of methodologies, including anti-CD14 coated beads or columns, or utilization of the phagocytotic activity of these cells to facilitate removal. Paramagnetic particles of a size sufficient to be engulfed by phagocytotic monocytes may be used. The paramagnetic particles may be commercially available beads, for example, those produced by Life Technologies under the trade name Dynabeads^{™}. Other non-specific cells may be removed by coating the paramagnetic particles with "irrelevant" proteins (e.g., serum proteins or antibodies). Irrelevant proteins and antibodies include those proteins and antibodies or fragments thereof that do not specifically target the T cells to be isolated. The irrelevant beads may include beads coated with sheep anti-mouse antibodies, goat anti-mouse antibodies, and human serum albumin.

In brief, such depletion of monocytes is performed by preincubating T cells isolated from whole blood, apheresed peripheral blood, or tumors with one or more varieties of irrelevant or non-antibody coupled paramagnetic particles at any amount that allows for removal of monocytes (approximately a 20:1 bead:cell ratio) for about 30 minutes to 2 hours at 22 to 37 degrees C., followed by magnetic removal of cells which have attached to or engulfed the paramagnetic particles. Such separation can be performed using standard methods available in the art. For example, any magnetic separation methodology can be used including a variety of which are commercially available, (e.g., DYNAL^{®} Magnetic Particle Concentrator (DYNAL MPC^{®})). Assurance of requisite depletion can be monitored by a variety of methodologies known to those of ordinary skill in the art, including flow cytometric analysis of CD14 positive cells, before and after depletion.

For isolation of a desired population of cells by positive or negative selection, the concentration of cells and surface (e.g., particles such as beads) can be varied. It can be desirable to significantly decrease the volume in which beads and cells are mixed together (i.e., increase the concentration of cells), to ensure maximum contact of cells and beads. A concentration of 2 billion cells/ml may be used. A concentration of 1 billion cells/ml may be used. Greater than 100 million cells/ml may be used. A concentration of cells of 10, 15, 20, 25, 30, 35, 40, 45, or 50 million cells/ml may be used. A concentration of cells from 75, 80, 85, 90, 95, or 100 million cells/ml may be used. Concentrations of 125 or 150 million cells/ml can be used. Using high concentrations can result in increased cell yield, cell activation, and cell expansion. Further, use of high cell concentrations allows more efficient capture of cells that can weakly express target antigens of interest, such as CD28-negative T cells, or from samples where there are many tumor cells present (i.e., leukemic blood, tumor tissue, etc). Such populations of cells can have therapeutic value and would be desirable to obtain. For example, using high concentration of cells allows more efficient selection of CD8+ T cells that normally have weaker CD28 expression.

It can be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. The concentration of cells used can be 5×10⁶/ml. The concentration used can be from about 1×10⁵/ml to 1×10⁶/ml, and any integer value in between.

T cells can also be frozen. The freeze and subsequent thaw step can provide a more uniform product by removing granulocytes and to some extent monocytes in the cell population. After a washing step to remove plasma and platelets, the cells can be suspended in a freezing solution. While many freezing solutions and parameters are known in the art and will be useful in this context, one method involves using PBS containing 20% DMSO and 8% human serum albumin, or other suitable cell freezing media, the cells then are frozen to -80° C at a rate of 1° per minute and stored in the vapor phase of a liquid nitrogen storage tank. Other methods of controlled freezing can be used as well as uncontrolled freezing immediately at -20° C. or in liquid nitrogen.

T cells for use in the present invention can also be antigen-specific T cells. For example, tumor-specific T cells can be used. Antigen-specific T cells can be isolated from a patient of interest, such as a patient afflicted with a melanoma, such as patient with a melanoma tumor. The patient may have melanoma.

Neoepitopes may be determined for a subject and T cells specific to these antigens are isolated. Antigen-specific cells for use in expansion can also be generated *in vitro* using any number of methods known in the art, for example, as described in U.S. Patent Publication No. US 20040224402 entitled, Generation And Isolation of Antigen-Specific T Cells, or in U.S. Pat. Nos. 6,040,177. Antigen-specific cells for use in the present invention can also be generated using any number of methods known in the art, for example, as described in Current Protocols in Immunology, or Current Protocols in Cell Biology, both published by John Wiley & Sons, Inc., Boston, Mass.

It can be desirable to sort or otherwise positively select (e.g. via magnetic selection) the antigen specific cells prior to or following one or two rounds of expansion. Sorting or positively selecting antigen-specific cells can be carried out using peptide-MHC tetramers (Altman, et al., Science. 1996 Oct. 4; 274(5284):94-6). The adaptable tetramer technology approach may be used (Andersen et al., 2012 Nat Protoc. 7:891-902). Tetramers are limited by the need to utilize predicted binding peptides based on prior hypotheses, and the restriction to specific HLAs. Peptide-MHC tetramers can be generated using techniques known in the art and can be made with any MHC molecule of interest and any antigen of interest as described herein. Specific epitopes to be used in this context can be identified using numerous assays known in the art. For example, the ability of a polypeptide to bind to MHC class I can be evaluated indirectly by monitoring the ability to promote incorporation of ¹²⁵I labeled β2-microglobulin (β2m) into MHC class I/β2m/peptide heterotrimeric complexes (see Parker et al., J. Immunol. 152:163, 1994).

The T cells may be recombinantly modified to express a modified or chimeric receptor (e.g., chimeric antigen receptor (CAR) modified T cells).

Cells may be directly labeled with an epitope-specific reagent for isolation by flow cytometry followed by characterization of phenotype and TCRs. T cells may be isolated by contacting the T cell specific antibodies. Sorting of antigen-specific T cells, or generally any cells of the present invention, can be carried out using any of a variety of commercially available cell sorters, including, but not limited to, MoFlo sorter (DakoCytomation, Fort Collins, Colo.), FACSAria^{™}, FACSArray^{™}, FACSVantage^{™}, BD^{™} LSR II, and FACSCalibur^{™} (BD Biosciences, San Jose, Calif.).

The method may comprise selecting cells that also express CD3. The method can comprise specifically selecting the cells in any suitable manner. Preferably, the selecting is carried out using flow cytometry. The flow cytometry can be carried out using any suitable method known in the art. The flow cytometry can employ any suitable antibodies and stains. Preferably, the antibody is chosen such that it specifically recognizes and binds to the particular biomarker being selected. For example, the specific selection of CD3, CD8, TIM-3, LAG-3, 4-1BB, or PD-1 can be carried out using anti-CD3, anti-CD8, anti-TIM-3, anti-LAG-3, anti-4-1BB, or anti-PD-1 antibodies, respectively. The antibody or antibodies can be conjugated to a bead (e.g., a magnetic bead) or to a fluorochrome. Preferably, the flow cytometry is fluorescence-activated cell sorting (FACS). TCRs expressed on T cells can be selected based on reactivity to autologous tumors. Additionally, T cells that are reactive to tumors can be selected for based on markers using the methods described in patent publication Nos. WO2014133567 and WO2014133568. Additionally, activated T cells can be selected for based on surface expression of CD107a.

The method may further comprise expanding the numbers of T cells in the enriched cell population. Such methods are described in U.S. Patent No. 8,637,307. The T cells can be expanded before or after treatment of the cells with the PTD-MYC polypeptide. The numbers of T cells can be increased at least about 3-fold (or 4-, 5-, 6-, 7-, 8-, or 9-fold), more preferably at least about 10-fold (or 20-, 30-, 40-, 50-, 60-, 70-, 80-, or 90-fold), more preferably at least about 100-fold, more preferably at least about 1,000 fold, or most preferably at least about 100,000-fold. The numbers of T cells can be expanded using any suitable method known in the art. Exemplary methods of expanding the numbers of cells are described in patent publication No. WO 2003057171, U.S. Patent No. 8,034,334, and U.S. Patent Application Publication No. 2012/0244133.

Ex *vivo* T cell expansion can be performed by isolation of T cells and subsequent stimulation or activation followed by further expansion. The T cells can be stimulated or activated by a single agent. T cells may be stimulated or activated with two agents, one that induces a primary signal and a second that is a co-stimulatory signal. Ligands useful for stimulating a single signal or stimulating a primary signal and an accessory molecule that stimulates a second signal can be used in soluble form. Ligands can be attached to the surface of a cell, to an Engineered Multivalent Signaling Platform (EMSP), or immobilized on a surface. Both primary and secondary agents can be co-immobilized on a surface, for example a bead or a cell. The molecule providing the primary activation signal can be a CD3 ligand, and the co-stimulatory molecule can be a CD28 ligand or 4-1BB ligand. The cells may be expanded by stimulation with one or more antigens, such as a melanoma tumor antigen or antigens derived from the patient's tumor.

The isolated immune cells may be immediately treated with the PTD-MYC fusion polypeptide following isolation. The isolated immune cells are stored in a suitable buffer and frozen prior to treatment with the PTD-MYC fusion polypeptide. The isolated immune cells may be immediately treated with the PTD-MYC fusion polypeptide following isolation and the treated cells are stored in a suitable buffer and frozen until needed for administration to the patient.

The isolated immune cells (e.g., a mixed population immune cells or isolated types, such as tumor infiltrating lymphocytes) may be contacted with a composition containing a PTD-MYC fusion polypeptide for a period of time sufficient to be taken up by the cells. The immune cells can be contacted with a composition containing a PTD-MYC fusion polypeptide for less than about 24 hours, less than about 23 hours, less than about 22 hours, less than about 21 hours, less than about 20 hours, less than about 19 hours, less than about 18 hours, less than about 17 hours, less than about 16 hours, less than about 15 hours, less than about 14 hours, less than about 13 hours, less than about 12 hours, less than about 11 hours, less than about 10 hours, less than about 9 hours, less than about 8 hours, less than about 7 hours, less than about 6 hours, less than about 5 hours, less than about 4 hours, less than about 3 hours, less than about 2 hours, or less than about 1 hour.

The immune cells can be contacted with a composition containing a PTD-MYC fusion polypeptide for less than about 55 minutes, less than about 50 minutes, less than about 45 minutes, less than about 40 minutes, less than about 35 minutes, less than about 30 minutes, less than about 29 minutes, less than about 28 minutes, less than about 27 minutes, less than about 26 minutes, less than about 25 minutes, less than about 24 minutes, less than about 23 minutes, less than about 22 minutes, less than about 21 minutes, less than about 20 minutes, less than about 19 minutes, less than about 18 minutes, less than about 17 minutes, less than about 16 minutes, less than about 15 minutes, less than about 14 minutes, less than about 13 minutes, less than about 12 minutes, less than about 11 minutes, or less than about 10 minutes. The immune cells can be contacted with a composition containing a PTD-MYC fusion polypeptide for about 1 hour.

The immune cells can be contacted with a composition containing a PTD-MYC fusion polypeptide for 24 hours or longer. The immune cells can be contacted with a composition containing a PTD-MYC fusion polypeptide for less than about 12 days, less than about 11 days, less than about 10 days, less than about 9 days, less than about 8 days, less than about 7 days, less than about 6 days, less than about 5 days, less than about 4 days, less than about 2 days, or less than about 1 day.

The cells may be contacted with a MYC-fusion polypeptide at a concentration of 0.5µg/ml to 500 µg/ml. 0.5 µg/ml, at least 0.6µg/ml, at least 0.7µg/ml, at least 0.8µg/ml, at least 0.9µg/ml, at least 1µg/ml, at least 2µg/ml, at least 3µg/ml, at least 4µg/ml, at least 5µg/ml, at least 6µg/ml, at least 7µg/ml, at least 8µg/ml, at least 9µg/ml, at least 10µg/ml, at least 15µg/ml, at least 20µg/ml, at least 25µg/ml, at least 30µg/ml, at least 35µg/ml, at least 40µg/ml, at least 45µg/ml, at least 50µg/ml, at least 55µg/ml, at least 60µg/ml, at least 65µg/ml, at least 70µg/ml, at least 75µg/ml, at least 80µg/ml, at least 85µg/ml, at least 90µg/ml, at least 95µg/ml, or at least 100µg/ml.

### MYC fusion proteins

The PTD-MYC fusion polypeptide may comprise a protein transduction domain (PTD), a MYC polypeptide that promotes one or more of cell survival or proliferation, and optionally a protein tag domain, *e.g*., one or more amino acid sequences that facilitate purification of the fusion protein. A cell contacted with MYC polypeptide may exhibit increased survival time (*e.g.,* as compared to an identical or similar cell of the same type that was not contacted with MYC), and/or increased proliferation (e.g., as compared to an identical or similar cell of the same type that was not contacted with MYC).

The fusion protein may comprise (a) a protein transduction domain; and (b) a MYC polypeptide sequence. The fusion peptide may be a peptide of Formula (I):
protein transduction domain-MYC polypeptide sequence.

A fusion peptide disclosed herein may comprise (a) a protein transduction domain; (b) a MYC polypeptide sequence; and (c) one or more molecules that link the protein transduction domain and the MYC polypeptide sequenceThe fusion peptide may be a peptide of Formula (II):
protein transduction domain-X-MYC polypeptide sequence,
wherein -X- is molecule that links the protein transduction domain and the MYC polypeptide sequence. -X- may be at least one amino acid.

A fusion peptide disclosed herein may comprise (a) a protein transduction domain; (b) a MYC polypeptide sequence; (c) at least two protein tags; and (d) optionally linker(s). The fusion peptide may be a peptide of Formula (III-VI):
protein transduction domain-X-MYC polypeptide sequence-X-protein tag 1-X-protein tag 2 (Formula (III)), or
protein transduction domain-MYC polypeptide sequence-X-protein tag 1-X-protein tag 2 (Formula (IV)), or
protein transduction domain-MYC polypeptide sequence-protein tag 1-X-protein tag 2 (Formula (V)), or
protein transduction domain-MYC polypeptide sequence-protein tag 1-protein tag 2 (Formula (VI)),
wherein -X- is a linker. -X- may be one or more amino acids.

A fusion peptide disclosed herein may comprise (a) a protein transduction domain; (b) a MYC polypeptide sequence; (c) a 6-histidine tag; (d) a V5 epitope tag: and (e) optionally linker(s). The fusion peptide may be a peptide of Formula (VII-XIV):
protein transduction domain-X-MYC polypeptide sequence-X-6-histidine tag-X-V5 epitope tag (Formula (VII)), or
protein transduction domain-MYC polypeptide sequence-X-6-histidine tag-X-V5 epitope tag (Formula (VIII)), or
protein transduction domain-MYC polypeptide sequence-6-histidine tag-X-V5 epitope tag (Formula (IX)), or
protein transduction domain-MYC polypeptide sequence-6-histidine tag-V5 epitope tag (Formula (X)),
protein transduction domain-X-MYC polypeptide sequence-X-V5 epitope tag-X-6-histidine tag (Formula (XI)), or
protein transduction domain-MYC polypeptide sequence-X-V5 epitope tag-X-6-histidine tag (Formula (XII)), or
protein transduction domain-MYC polypeptide sequence-V5 epitope tag-X-6-histidine tag (Formula (XIII)), or
protein transduction domain-MYC polypeptide sequence-V5 epitope tag-6-histidine tag (Formula (XIV)),
wherein -X- is a linker. -X- may be one or more amino acids.

As noted above, the MYC fusion protein may comprise one or more linker sequences. The linker sequences can be employed to link the protein transduction domain, MYC polypeptide sequence, V5 epitope tag and/or 6-histidine tag of the fusion protein. The linker may comprise one or more amino acids. The amino acid sequence of the linker may comprise KGELNSKLE. The linker may comprise the amino acid sequence of RTG.

### Protein Transduction Domain (PTD)

The MYC fusion protein may include a protein transduction domain. Peptide transport provides an alternative for delivery of small molecules, proteins, or nucleic acids across the cell membrane to an intracellular compartment of a cell. One non-limiting example and well-characterized protein transduction domain (PTD) is a TAT-derived peptide. Frankel et al., (see, e.g., U.S. Pat. No. 5,804,604, U.S. Pat. No. 5,747,641, U.S. Pat. No. 5,674,980, U.S. Pat. No. 5,670,617, and U.S. Pat. No. 5,652,122) demonstrated transport of a cargo protein (β-galactosidase or horseradish peroxidase) into a cell by conjugating a peptide containing amino acids 48-57 of TAT to the cargo protein. TAT may comprise an amino acid sequence of MRKKRRQRRR (SEQ ID NO: 7).

Another non-limiting example of a PTD is penetratin. Penetratin can transport hydrophilic macromolecules across the cell membrane (Derossi et al., Trends Cell Biol., 8:84-87 (1998)). Penetratin is a 16 amino acid peptide that corresponds to amino acids 43-58 of the homeodomain of Antennapedia, a *Drosophila* transcription factor which is internalized by cells in culture.

Yet another non-limiting example of a PTD is VP22. VP22, a tegument protein from Herpes simplex virus type 1 (HSV-1), has the ability to transport proteins and nucleic acids across a cell membrane (Elliot et al., Cell 88:223-233, 1997). Residues 267-300 of VP22 are necessary but cannot be sufficient for transport. Because the region responsible for transport function has not been identified, the entire VP22 protein is commonly used to transport cargo proteins and nucleic acids across the cell membrane (Schwarze et al., Trends Pharmacol Sci, 21:45-48, 2000).

The PTD-MYC fusion polypeptide may include a protein transduction domain. By way of example, but not by way of limitation, the protein transduction domain may comprise the protein transduction domain of one or more of TAT, penetratin, VP22, vpr, EPTD, R9, R15, VP16, and Antennapedia. The protein transduction domain may comprise the protein transduction domain of one or more of TAT, penetratin, VP22, vpr, and EPTD. The protein transduction domain may comprise the protein transduction domain of at least one of TAT, penetratin, VP22, vpr, EPTD, R9, R15, VP16, and Antennapedia. The protein transduction domain may comprise a synthetic protein transduction domain (e.g., polyarginine or PTD-5). The protein transduction domain may comprise a TAT protein transduction domain. The protein transduction domain may be covalently linked to the MYC polypeptide. The protein transduction domain may be linked to the MYC polypeptide via a peptide bond. The protein transduction domain may be linked to the MYC polypeptide via a linker sequence. The linker may comprise a short amino acid sequence. By way of example, but not by way of limitation, the linker sequences may be 1, 2, 3, 4, 5, 6, 7, 8, 9, or 10 amino acids in length.

The MYC fusion protein of the present technology can be arranged in any desired order. The MYC fusion protein can be arranged in order of a) the protein transduction domain linked in frame to the MYC polypeptide, b) the MYC polypeptide linked in frame to the V5 domain, and c) the V5 domain linked in frame to the 6-histidine epitope tag. The MYC fusion protein may have an order of components of a) the MYC polypeptide linked in frame to the protein transduction domain, b) the protein transduction domain linked in frame to the V5 domain, and c) the V5 domain linked in frame to the 6-histidine epitope tag. Additional amino acid sequences can be included between each of the sequences. Additional amino acids can be included at the start and/or end of the polypeptide sequences.

The protein transduction domain may be a TAT protein transduction domain. The protein transduction domain may be TAT_{[48-57]}. The protein transduction domain may be TAT_{[57-48]}.

### Protein Tag Domains

The MYC fusion protein maycomprise a protein tag domain that comprises one or more amino acid sequences that facilitate purification of the fusion protein. The protein tag domain may comprise one or more of a polyhistidine tag, and an epitope tag. By way of example, but not by way of limitation, exemplary tags include one or more of a V5, a histidine-tag (*e.g*., a 6-histidine tag), HA (hemagglutinin) tags, FLAG tag, CBP (calmodulin binding peptide), CYD (covalent yet dissociable NorpD peptide), Strepll, or HPC (heavy chain of protein C). The protein tag domain may comprise about 10 to 20 amino acids in length. The protein tag domain may comprise 2 to 40 amino acids in length, for example 6-20 amino acids in length. Two of the above listed tags (for example, V5 and the HIS-tag) may be used together to form the protein tag domain.

The histidine tag may be a 6-histidine tag. The histidine tag may comprise the sequence HHHHHH (SEQ ID NO:8). The fusion peptide disclosed herein may comprise a V5 epitope tag. The V5 tag may comprise the amino acid sequence of: GKPIPNPLLGLDST (SEQ ID NO:9). The V5 tag may comprise the amino acid sequence of IPNPLLGLD (SEQ ID NO:10).

The protein tags can be added to the fusion protein disclosed herein by any suitable method. By way of example, but not by way of limitation, a TAT-MYC polypeptide sequence may be cloned into an expression vector encoding one or more protein tags, *e.g.,* a polyHis-tag and/or a V5 tag. A polyhistidine tag and/or a V5 tag may be added by PCR (*i.e.,* the PCR primers comprise a polyhistidine sequence and/ or V5 sequence).

### Construction of PTD-MYC fusion polypeptides

PTD-MYC fusion polypeptides (*e.g*., TAT-MYC fusion polypeptide) disclosed herein can be constructed by methods well known in the art. By way of example, but not by way of limitation, a nucleotide sequence encoding a TAT-MYC fusion polypeptide can be generated by PCR. A forward primer for a human MYC sequence may comprise an in frame N-terminal 9-amino-acid sequence of the TAT protein transduction domain (*e.g*., RKKRRQRRR). A reverse primer for a human MYC sequence may be designed to remove the stop codon. The PCR product may be cloned into any suitable expression vector. The expression vector may comprise a polyhistidine tag and a V5 tag.

A fusion peptide disclosed herein may comprise (a) TAT, and (b) c-MYC . A fusion peptide disclosed herein may comprise (a) TAT_{[48-57]}, and (b) c-MYC . A fusion peptide disclosed herein may comprise (a) TAT_{[57-48]}, and (b) c-MYC .

A fusion peptide disclosed herein may comprise (a) TAT, (b) c-MYC , (c) linker(s), (d) V5 tag, and (e) 6-histidine tag. Afusion peptide disclosed herein may comprise (a) TAT_{[48-57]}, (b) c-MYC , (c) linker(s), (d) V5 tag, and (e) 6-histidine tag. A fusion peptide disclosed herein may comprise (a) TAT_{[57-48]}, (b) c-MYC , (c) linker(s), (d) V5 tag, and (e) 6-histidine tag.

The PTD-MYC fusion polypeptide may comprise SEQ ID NO: 1; the PTD-MYC fusion polypeptide may be SEQ ID NO: 1.

The fusion protein can be modified during or after synthesis to include one or more functional groups. By way of example but not by way of limitation, the protein can be modified to include one or more of an acetyl, phosphate, acetate, amide, alkyl, and/or methyl group. This list is not intended to be exhaustive, and is exemplary only. The protein may include at least one acetyl group.

A PTD-MYC fusion polypeptide can be generated by any suitable method known the art, e.g. by recombinant protein expression in a cell, such as a bacterial cell, an insect cell, or mammalian cell. A PTD-MYC fusion polypeptide may be recombinantly produced by microbial fermentation. Microbial fermentation may be performed in a fermentation volume of from about 1 to about 10,000 liters, for example, a fermentation volume of about 10 to about 1000 liters. The fermentation can utilize any suitable microbial host cell and culture medium. *E. coli* may be utilized as the microbial host cell. Other microorganisms can be used, *e.g., S. cerevisiae, P. pastoris, Lactobacilli, Bacilli* and *Aspergilli.* In The microbial host cell may be BL-21 Star^{™} *E*. *coli* strain (Invitrogen). The microbial host cell may be BLR DE3 *E. coli.* strain.

The host cells may be modified to provide tRNAs for rare codons, which are employed to overcome host microbial cell codon bias to improve translation of the expressed proteins. The host cells (*e.g., E. coli*) may be transformed with a plasmid, such as pRARE (CamR), which express tRNAs for AGG, AGA, AUA, CUA, CCC, GGA codons. Additional, suitable plasmids or constructs for providing tRNAs for particular codons are known in the art and can be employed in the methods provided.

Integrative or self-replicative vectors can be used for the purpose of introducing the PTD-MYC fusion polypeptide expression cassette into a host cell of choice. In an expression cassette, the coding sequence for the PTD-MYC fusion polypeptide is operably linked to promoter, such as an inducible promoter. Inducible promoters are promoters that initiate increased levels of transcription from DNA under their control in response to some change in culture conditions, *e.g*., the presence or absence of a nutrient or a change in temperature. The nucleic acid encoding the PTD-MYC fusion polypeptide may be codon optimized for bacterial expression.

Exemplary promoters that are recognized by a variety of potential host cells are well known. These promoters can be operably linked to PTD-MYC fusion polypeptide-encoding DNA by removing the promoter from the source DNA, if present, by restriction enzyme digestion and inserting the isolated promoter sequence into the vector. Promoters suitable for use with microbial hosts include, but are not limited to, the β-lactamase and lactose promoter systems (Chang et al., (1978) Nature, 275:617-624; Goeddel et al., (1979) Nature, 281: 544), alkaline phosphatase, a tryptophan (trp) promoter system (Goeddel (1980) Nucleic Acids Res. 8: 4057; EP 36,776), and hybrid promoters such as the tac promoter (deBoer et al., (1983) Proc. Natl. Acad. Sci. USA 80: 21-25). Any promoter for suitable for expression by the selected host cell can be used. Nucleotide sequences for suitable are published, thereby enabling a skilled worker operably to ligate them to DNA encoding PTD-MYC fusion polypeptide (see, *e.g.,* Siebenlist et al., (1980) Cell 20: 269) using linkers or adaptors to supply any required restriction sites. Promoters for use in bacterial systems can contain a Shine-Dalgarno (S.D.) sequence operably linked to the coding sequence. The inducible promoter may be the lacZ promoter, which is induced with Isopropyl β-D-1-thiogalactopyranoside (IPTG), as is well-known in the art. Promoters and expression cassettes can also be synthesized *de novo* using well known techniques for synthesizing DNA sequences of interest. The expression vector for expression of the PTD-MYC fusion polypeptides herein may be pET101/D-Topo (Invitrogen).

For expression of the PTD-MYC fusion polypeptides, the microbial host containing the expression vector encoding the PTD-MYC fusion polypeptide is typically grown to high density in a fermentation reactor. The reactor may be controlled feeds for glucose. A fermenter inoculum may first be cultured in medium supplemented with antibiotics (*e.g*., overnight culture). The fermenter inoculum is then used to inoculate the fermenter culture for expression of the protein. At an OD600 of at least about 15, usually at least about 20, at least 25, at least about 30 or higher, of the fermenter culture, expression of the recombinant protein is induced. Where the inducible promoter is the lacZ promoter, IPTG may be added to the fermentation medium to induce expression of the PTD-MYC fusion polypeptide. Generally, the IPTG is added to the fermenter culture at an OD600 which represents logarithmic growth phase.

Induced protein expression may be maintained for around about 2 to around about 5 hours post induction, and can be from around about 2 to around about 3 hours post-induction. Longer periods of induction may be undesirable due to degradation of the recombinant protein. The temperature of the reaction mixture during induction is preferably from about 28°C to about 37°C, usually from about 30°C to about 37°C. Induction may be at about 37°C.

The PTD-MYC fusion polypeptide is typically expressed as cytosolic inclusion bodies in microbial cells. To harvest inclusion bodies, a cell pellet is collected by centrifugation of the fermentation culture following induction, frozen at -70°C or below, thawed and resuspended in disruption buffer. The cells are lysed by conventional methods, *e.g*., sonication, homogenization, etc. The lysate is then resuspended in solubilization buffer, usually in the presence of urea at a concentration effective to solubilize proteins, *e.g*., from around about 5M, 6M, 7M, 8M, 9M or greater. Resuspension may require mechanically breaking apart the pellet and stirring to achieve homogeneity. The cell pellet may be directly resuspended in urea buffer and mixed until homogenous. The resuspension/solubilization buffer may be 8M Urea, 50 mM Phosphate pH 7.5 and the suspension may be passed through a homogenizer.

The homogenized suspension may be sulfonylated. The homogenized suspension may be adjusted to include 200 mM Sodium Sulfite and 10 mM Sodium Tetrathionate. The solution is then mixed at room temperature until homogeneous. The mixed lysate is then mixed for an additional period of time to complete the sulfonylation (*e.g*., at 2-8°C for ≥ 12 hours). The sulfonylated lysate was then centrifuged for an hour. The supernatant containing the sulfonylated PTD-MYC fusion polypeptides is then collected by centrifugation and the cell pellet discarded. The supernatant is then passed through a filter, *e.g.,* 0.22 µm membrane filter to clarify the lysate.

The solubilized protein is then purified. Purification methods may include affinity chromatography, reverse phase chromatography, gel exclusion chromatography, and the like. Affinity chromatography may be used. For example, the protein is provided with an epitope tag or histidine 6 tag for convenient purification. In the present methods, exemplary PTD-MYC fusion polypeptide comprise histidine 6 tag for purification using Ni affinity chromatography using Ni- resin.

The Ni- resin column may be equilibrated in a buffer containing urea. The equilibration buffer may be 6M Urea, 50 mM Phosphate, 500 mM NaCl, and 10% Glycerol solution. The sulfonylated and clarified supernatant comprising the PTD-MYC fusion polypeptide is then loaded onto the Ni- resin column. The column is then washed with a wash buffer, *e.g.,* 6M Urea, 50mM Phosphate, 10% Glycerol, 500 mM NaCl, pH 7.5. The column was then washed with sequential wash buffers with decreasing salt concentration. For example, exemplary subsequent washed can include 6M Urea, 50mM Phosphate, 10% Glycerol, and 2M NaCl, pH 7.5, followed another wash of 6M Urea, 50mM Phosphate, 10% Glycerol, 50mM NaCl, and 30mM Imidazole, pH 7.5.

Following sequential application of the wash buffers the PTD-MYC fusion polypeptide is eluted from the column by addition of elution buffer, *e.g.,* 6M Urea, 50mM Phosphate, 10% Glycerol, and 50mM NaCl, pH 7.5 with a gradient from 100 to 300 mM Imidazole, and collecting fractions. The protein containing fractions to be pooled are then filtered through a 0.22 µm membrane. Assessment of protein yield can be measured using any suitable method, *e.g*., spectrophotometry at UV wavelength 280.

One or more additional purification methods can be employed to further purify the isolated PTD-MYC fusion polypeptides. The pooled fractions from the Ni-Sepharose chromatography step can be further purified by anion exchange chromatography using a Q-Sepharose resin. The pool may be prepared for loading onto the Q-Sepharose column by diluting the samples to the conductivity of the Q sepharose buffer (17.52 +/-1 mS/cm) with the second wash buffer (*e.g.,* 6M Urea, 50mM Phosphate, 10% Glycerol, 2M NaCl, pH 7.5) from the Ni Sepharose chromatography step. The diluted pool is then loaded onto the Q-Sepharose column, followed by two chase steps using a chase buffer (*e.g.,* 6M Urea, 50mM Phosphate, 300mM NaCl, and 10% Glycerol), with further sequential applications of the chase buffer until the UV trace reaches baseline, indicating that the protein has eluted from the column.

### Methods of Treatment

The PTD-MYC fusion polypeptide-modified immune cells are administered for the treatment of a melanoma in a patient. The patient may have a metastatic melanoma. The patient may have received one or more agents for the treatment of the melanoma prior to administration of the PTD-MYC fusion polypeptide-modified immune cells. The melanoma may be a relapsed or refractory melanoma. The melanoma may be a metastatic melanoma. The melanoma may be a superficial spreading melanoma, a nodular melanoma, a lentigo maligna melanoma, or an acral melanoma. The melanoma may be resistant to one or more agents for the treatment of the melanoma.

Administration of the PTD-MYC fusion polypeptide-modified immune cells may inhibit growth of a melanoma tumor or reduces the volume of a melanoma tumor. Administration of the PTD-MYC fusion polypeptide-modified immune cells to a subject having a melanoma may alleviate one or more symptoms of the melanoma. Administration of the PTD-MYC fusion polypeptide-modified immune cells to a subject having melanoma may increase the overall survival of the subject. Administration of the PTD-MYC fusion polypeptide-modified immune cells to a subject having melanoma may increase the regression of the melanoma.

The administration of the PTD-MYC fusion polypeptide-modified immune cells (e.g. PTD-MYC fusion polypeptide treated tumor infiltrating lymphocytes) according to the methods provided herein can be carried out in any suitable manner for administering cells to a subject, including but not limited to injection, transfusion, implantation or transplantation. The PTD-MYC fusion polypeptide-modified immune cells may be administered to a patient subcutaneously, intradermally, intratumorally, intranodally, intramedullary, intramuscularly, intrathecally, by intravenous or intralymphatic injection, or intraperitoneally. The PTD-MYC fusion polypeptide-immune cells may be administered into a cavity formed by the resection of tumor tissue (i.e. intracavity delivery) or directly into a tumor prior to resection (i.e. intratumoral delivery). The MYC-fusion polypeptide-immune cells may be administered by intravenous injection.

In addition to the PTD-MYC fusion polypeptide-modified immune cells, compositions for administration can comprise any other agents such as pharmaceutically acceptable carriers, buffers, excipients, adjuvants, additives, antiseptics, filling, stabilizing and/or thickening agents, and/or any components normally found in corresponding products. Selection of suitable ingredients and appropriate manufacturing methods for formulating the compositions for particular routes of administration generally known in the art.

The adoptive cell therapeutic composition comprising PTD-MYC fusion polypeptide-modified immune cells can be in any form, such as solid, semisolid or liquid form, suitable for administration. A formulation can be selected from a group consisting of, but not limited to, solutions, emulsions, suspensions, tablets, pellets and capsules. The compositions are not limited to a certain formulation, instead the composition can be formulated into any known pharmaceutically acceptable formulation. The pharmaceutical compositions may be produced by any conventional processes known in the art.

The administration of the MYC-fusion polypeptide-modified immune cells may comprise administering of 10⁴-10¹⁰ of the cells per kg body weight, including 10⁵ to 10⁶ cells/kg body weight, including all integer values of cell numbers within those ranges. The cells may be administered with or without a course of lymphodepletion, for example with cyclophosphamide.

The MYC-fusion polypeptide-modified immune cells can be administrated in one or more doses. The therapeutically effective amount of PTD-MYC fusion polypeptide-modified immune cells may be administrated as a single dose. Administering a single dose of the PTD-MYC fusion polypeptide-modified immune cells may have a therapeutic effect. The effective amount of MYC-fusion polypeptide-modified immune cells may be administrated as more than one dose over a period time. Timing of administration is within the judgment of managing physician and depends on various factors, including, but not limited to the age, gender, or clinical condition of the patient and characteristics of the melanoma, including type, degree or location of melanoma. While individual needs vary, determination of optimal ranges of effective amounts of a MYC-fusion polypeptide-modified immune cell for treatment of a particular disease or conditions are within the skill of one in the art.

PTD-MYC fusion polypeptide-modified immune cells can be administered for example from 1 to 10 times in the first 2 weeks, 3 weeks, 4 weeks, monthly or during the treatment period. PTD-MYC fusion polypeptide-modified immune cells may be administered 2, 3, 4, 5, 6, 7, 8, 9, or 10 times. PTD-MYC fusion polypeptide-modified immune cells may be administered weekly, every 2 weeks, every 3 weeks or monthly.

A therapeutically effective amount means an amount which provides a therapeutic or prophylactic benefit. The dosage administrated will be dependent upon the age, health and weight of the recipient, kind of concurrent treatment, if any, frequency of treatment and the nature of the effect desired.

A patient receiving PTD-MYC modified immune cells may be first pretreated with one or more cytokines and/or other immunomodulatory agents. A patient receiving PTD-MYC modified immune cells may be lymphodepleted prior to administration of the PTD-MYC modified immune cells. The purpose of lymphodepletion is to make room for the infused lymphocytes, in particular by eliminating regulatory T cells and other non-specific T cells which compete for homeostatic cytokines.

The PTD-MYC modified immune cells may be administered with an additional therapeutic agent. Additional therapeutic agent may be administered prior to, simultaneously with, intermittently with, or following treatment with the PTD-MYC modified immune cells. The additional therapeutic agent may be an immunomodulator, such as an interleukin (e.g. IL-2, IL-7, IL-12), a cytokine, a chemokine, or and immunomodulatory drug. The cytokine may be selected from among cytokine is selected from a group consisting of interferon alpha, interferon beta, interferon gamma, complement C5a, IL-2, TNFα, CD40L, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5 (=RANTES), CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 and XCL2. The additional therapeutic agent may be an anticancer agent, such as chemotherapy or radiation therapy.

The modified immune cells administered for the treatment of melanoma may be T cells with genetically modified antigen receptors, including chimeric antigen receptor (CAR)-T cells. Various strategies can, for example, be employed to genetically modify T cells by altering the specificity of the T cell receptor (TCR), for example, by introducing new TCR α and β chains with selected peptide specificity (see, e.g., U.S. Patent No. 8,697,854; PCT Patent Publications: WO2003020763, WO2004033685, WO2004044004, WO2005114215, WO2006000830, WO2008038002, WO2008039818, WO2004074322, WO2005113595, WO2006125962, WO2013166321, WO2013039889, WO2014018863, WO2014083173; U.S. Patent No. 8,088,379). Chimeric antigen receptors (CARs) can be used in order to generate immunoresponsive cells, such as T cells, specific for selected targets, such as malignant cells, with a wide variety of receptor chimera constructs having been described (*see,* e.g. U.S. Patent Nos. 5,843,728; 5,851,828; 5,912,170; 6,004,811; 6,284,240; 6,392,013; 6,410,014; 6,753,162; 8,211,422; and, PCT Publication WO9215322). Methods for the preparation of CAR T cells are known in the art and can be used in combination with the methods provided herein to generate modified CAR T cells comprising a MYC fusion polypeptide (e.g. PTD) as described herein.

In general, CARs are comprised of an extracellular domain, a transmembrane domain, and an intracellular domain, wherein the extracellular domain comprises an antigen-binding domain that is specific for a predetermined target. While the antigen-binding domain of a CAR is often an antibody or antibody fragment (e.g., a single chain variable fragment, scFv), the binding domain is not particularly limited so long as it results in specific recognition of a target. The antigen-binding domain may comprise a receptor, such that the CAR is capable of binding to the ligand of the receptor. Alternatively, the antigen-binding domain may comprise a ligand, such that the CAR is capable of binding the endogenous receptor of that ligand.

The T cells expressing a desired CAR may be selected through co-culture with γ-irradiated activating and propagating cells (AaPC), which co-express the melanoma antigen and co-stimulatory molecules. The engineered CAR T-cells can be expanded, for example by co-culture on AaPC in presence of soluble factors, such as IL-2 and IL-21. This expansion can for example be carried out so as to provide memory CAR+ T cells. In this way, CAR T cells can be provided that have specific cytotoxic activity against antigen-bearing tumors (optionally in conjunction with production of desired chemokines such as interferon-y).

TheCAR T-cells may then be contacted with a PTD-MYC fusion polypeptide provided herein *in vitro* to generation a modified CAR T cells for the treatment of a melanoma. The modified CAR T cells can be administered according any suitable method, including the methods for administration of the PTD-MYC fusion polypeptide-modified immune cells as described above.

### Kits

Pharmaceutical compositions comprising MYC-fusion polypeptides and/or MYC-fusion polypeptide-modified immune cells provided herein can be assembled into kits or pharmaceutical systems for use in treating a melanoma. Kits can comprise a carrier means, such as a box, carton, tube, having in close confinement therein one or more containers, such as vials, tubes, ampoules, bottles, syringes, or bags. The kits can also comprise associated instructions for using the MYC-fusion polypeptides and/or MYC-fusion polypeptide-modified immune cells.

The kit may comprise an effective amount of an adoptive cell therapy, such as MYC-fusion polypeptide-modified immune cells. Thekit may comprise one for more reagents for the detection of the administered MYC-fusion polypeptides and/or MYC-fusion polypeptide-modified immune cells. The kit may comprise cells for treatment with a MYC-fusion polypeptide provided herein, for example, hematopoietic stem cells, donor leukocytes, T cells, or NK cells. Thekit may further comprise an effective amount of a therapeutic agent to be administered in combination with MYC-fusion polypeptides and/or MYC-fusion polypeptide-modified immune cells provided herein. Therapeutic agent may be an anti-cancer agent.

Kits provided herein also can include a device for administering MYC-fusion polypeptides and/or MYC-fusion polypeptide-modified immune cells provided herein to a subject. Any of a variety of devices known in the art for administering polypeptides and cells to a subject can be included in the kits provided herein. Exemplary devices include a hypodermic needle, an intravenous needle, a catheter, a needle-less injection, but are not limited to, a hypodermic needle, an intravenous needle, a catheter, a needle-less injection device, an inhaler and a liquid dispenser such as an eyedropper. Typically the device for administering the MYC-fusion polypeptides and/or MYC-fusion polypeptide-modified immune cells of the kit will be compatible with the desired method of administration of the composition. For example, a composition to be delivered intravenously can be included in a kit with a hypodermic needle and a syringe.

### EXAMPLES

### Example 1. Immune cells treated with TAT-MYC to generate TAT-MYC-treated lymphocytes for immunotherapy of melanoma tumors

In this example, the ability of a PTD-MYC fusion polypeptide comprising the protein transduction domain of HIV-1 transactivation protein (TAT) and MYC to modulate an immune response against melanoma cells *in vivo* was examined. Specifically, the ability of lymphoid cells, derived from melanoma-bearing mice and treated with TAT-MYC, to treat mice harboring melanoma tumors was studied. The object of these studies was to determine whether immune cells derived from melanoma bearing mice and treated with TAT-MYC to generate TAT-MYC lymphocytes would be an effective treatment for melanoma tumors upon transplantation into melanoma bearing mice.

### Materials and Methods

C57BL/6J is the most widely used inbred strain and the first to have its genome sequenced. Although this strain is refractory to many tumors, it is a permissive background for maximal expression of most mutations. C57BL/6J mice are resistant to audiogenic seizures, have a relatively low bone density, and develop age-related hearing loss. They are also susceptible to diet-induced obesity, type 2 diabetes, and atherosclerosis. Macrophages from this strain are resistant to the effects of anthrax lethal toxin.

### Treatment Groups

Fifteen C57BL/6 mice (Jackson Laboratory Stock# 000664) weighing approximately 25g and harboring melanoma tumors were generated and divided into 3 cohorts of 5 animals, one cohort of one mouse as a no treatment control, one cohort treated with Lymphoid cells derived from tumor-bearing mice and treated with control TAT-fusion protein, and one cohort treated with TAT-MYC lymphocytes.

### Generation of tumor-bearing donor mice and preparation of donor cells

B16-F10 melanoma cells (ATCC CRL 6475, mouse skin melanoma) for implantation were cultured in D10 media (DMEM, 10% FBS, Pen/Strep (10,000 units per/ml) (Gibco Cat# 15140); L-glutamine (200mM) (Gibco Cat# 25030); MEM Non-essential Amino Acids (Gibco Cat# 11140)).

The C57BL/6j mice (Jackson Laboratory #003548) were implanted with 1×10⁴ B16-F10 melanoma cells in 250 µL PBS via tail vein injection. Prior to injection, each test mouse was placed under a 250W heat lamp for 1-2 minutes and then injected intravenously with the melanoma cells. At 14 days post-transplant, lymph nodes from the injected mice were harvested and ground with the plunger of a 10 mL syringe.

For the first study, lymph nodes were harvested from 5 mice. For the second lymph nodes were harvested from 10 mice. The cells were washed with C10, collected and spun at 260 × g for 5 min. After discarding the supernatant, the cells were resuspended in 10mL sterile TAC, spun at 260 x g for 5 minutes. After discarding the supernatant, the cells were resuspended in 2mL of sterile filtered PBS with 5% BSA.

The lymph node cells were treated with TAT-MYC to generate TAT-MYC lymphocytes or treated with a control TAT-Fusion protein. The cells were split into 2, 15mL conical tubes (1mL each), treated with 1mL of 25ug/ml of a control protein (TAT-CRE for experiment 1, TAT-GFP for experiment 2) or 1mL of 25ug/ml of TAT-MYC lot C18. After one hour of room temp incubation, each tube was washed with sterile PBS three times, transferred to 5mL sterile tubes and placed on ice.

The test mice were prepared by injecting 1×10⁴ B16-F10 melanoma cells in 250uL PBS into the tail vein for each cohort of 5 C57BL/6j mice. After injection, the mice were observed once daily. Changes in body weight, food consumption, activity, and mortality were monitored. At 7 days post-transplant, TAT-MYC lymphocytes or control lymphoid cells were then transplanted into melanoma cell injected mice.

Symptoms were monitored daily. The mice were euthanized when severe symptoms presented and deaths were recorded. Mice were either found dead or euthanized if found with severe symptoms such as heavy breathing, hunched back and immobility. Day of death was recorded with day of treatment as Day 0.

The results from Experiments 1 and 2 are shown in Figures 1 and 2, respectively. As shown in the figures, treating melanoma-bearing mice with TAT-MYC lymphocytes (TBX-3400) generated by contacting mouse lymphoid cells derived from melanoma bearing mice with TAT-MYC, significantly improved the overall survival of the mice compared to transplanting lymphoid cells treated with control TAT-Fusion protein. These results suggest that TAT-MYC treatment of immune cells are useful in the treatment of melanoma using adoptive cell transfer.

### Example 2. Dose Response Effect of TAT-MYC-treated lymphocytes for immunotherapy of melanoma tumors

In this example, the therapeutic effects of different amounts administered TAT-MYC-treated lymphocytes for immunotherapy of melanoma tumors was examined. This experiment was performed as described above in Example 1, except that several different doses of the TAT-MYC-treated lymphocytes were injected and compared. Two experiments were performed. In the first experiment, Experiment 3, TAT-MYC lymphocytes were administered to the melanoma-bearing mice via tail vein injection according to the following dosing groups: 3.0×10⁶ cells/kg, 6.0×10⁶ cells/kg, 14.0×10⁶ cells/kg, and 70.0×10⁶ cells/kg. For the control groups, the mice were administered 70.0×10⁶ TAT-Cre treated or no cells (NT). In the second experiment, Experiment 4, TAT-MYC lymphocytes were administered to the melanoma-bearing mice via tail vein injection according to the following dosing groups: 4.0×10³ cells/kg, 4.0×10⁴ cells/kg, 4.0×10⁵ cells/kg, 4.0×10⁶ cells/kg and 4.0×10⁷ cells/kg. For the control groups, the mice were administered 4.0×10⁶ TAT-Cre treated or no cells (NT). The results from Experiments 3 and 4 are shown in Figures 3 and 4, respectively. As shown in the figures, treating melanoma-bearing mice with increasing amounts of TAT-MYC lymphocytes (TBX-3400) led to a significantly improved overall survival rate in both experiments. These experiments demonstrate the both the reproducibility and efficacy of TAT-MYC lymphocytes for treating melanoma-bearing subjects.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein *may* be employed in practicing the disclosure. It is intended that the following claims define the scope of the disclosure and that methods and structures within the scope of these claims and their equivalents be covered thereby.

Other embodiments are set forth within the following claims.

## Claims

1. A composition for use in adoptive cell therapy for treating a melanoma, wherein the composition comprises:
(a) a MYC fusion peptide, comprising (i) a protein transduction domain, optionally a TAT protein transduction domain sequence; (ii) a MYC polypeptide sequence; and
(b) one or more primary immune cells isolated from a donor subject that has a melanoma tumor, wherein the one or more primary immune cells are reactive against a melanoma-specific antigen.

2. The composition for the use of claim 1, wherein the MYC fusion peptide comprises SEQ ID NO: 1.

3. The composition for the use of any one of claims 1-2, wherein the one or more immune cells comprise one or more lymphocytes, optionally, a T cell, a B cell, an NK cell, or any combination thereof.

4. The composition for the use of claim 3, wherein the one or more lymphocytes comprise a tumor-infiltrating lymphocyte or are recombinantly modified to express a modified receptor or chimeric antigen receptor.

5. A modified immune cell for use in adoptive cell therapy for treating a melanoma in a subject wherein the modified immune cell comprises a MYC fusion peptide comprising (i) a protein transduction domain, optionally a TAT protein transduction domain sequence; and (ii) a MYC polypeptide sequence and is reactive against a tumor-specific antigen.

6. The modified immune cell for the use of claim 5 wherein the MYC fusion peptide comprises SEQ ID NO: 1; and/or wherein the one or more immune cells comprise one or more lymphocytes, optionally, a T cell, a B cell, an NK cell, or any combination thereof, and optionally wherein the one or more lymphocytes comprise a tumor-infiltrating lymphocyte or are recombinantly modified to express a modified receptor or chimeric antigen receptor.

7. The modified immune cell for the use of claim 5 or 6, wherein the one or more modified immune cells are derived from primary immune cells isolated from the recipient subject or isolated from a separate donor subject having the same type of melanoma.

8. The modified immune cell for the use of any one of claims 5-7, wherein the one or more modified immune cells are prepared by contacting the primary immune cells *in vitro* with the MYC fusion peptide following isolation from the donor subject.

9. The modified immune cell for the use of any one of claims 7-8, further comprising expanding the primary immune cells *in vitro* prior to or following contacting with the MYC fusion peptide, wherein the cells are optionally, expanded using an anti-CD3 antibody, irradiated allogenic feeder cells, or an exogenous cytokine, wherein the cytokine is optionally, interleukin-2.

10. The modified immune cell for the use of any one of claims 5-9, wherein the recipient subject is:
(i) lymphodepleted prior to administration of the one or more modified immune cells;
(ii) has been administered a cytokine prior to, during, or subsequent to administration of the one or more modified immune cells, wherein the cytokine is optionally selected from a group consisting of interferon α, interferon β, interferon γ, complement C5a, IL-2, TNFalpha, CD40L, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 and XCL2; and/or
(iii) is a human or a non-human animal.

11. The modified immune cell for the use of any one of claims 5-10, wherein the melanoma is metastatic.

12. The modified immune cell for the use of any one of claims 5-11, wherein the recipient subject has been administered an additional cancer therapy, optionally selected from among chemotherapy, radiation therapy, immunotherapy, monoclonal antibodies, anti-cancer nucleic acids or proteins, anti-cancer viruses or microorganisms, and any combinations thereof.

13. A method for preparing modified immune cells for adoptive cell therapy for melanoma, comprising contacting one or more immune cells *in vitro* with a MYC fusion polypeptide, wherein the immune cells are from a donor that has been exposed to one or more tumor antigens and wherein the MYC fusion peptide comprises (i) a protein transduction domain, optionally, a TAT protein transduction domain sequence; (ii) a MYC polypeptide sequence and are reactive to a tumor-specific antigen.

14. The method of claim 13, wherein the one or more modified immune cells are derived from primary immune cells isolated from a subject having melanoma.

15. The method of any one of claims 13-14, further comprising expanding the primary immune cells *in vitro* prior to or following contacting with the MYC fusion peptide; and/or wherein the MYC fusion peptide comprises SEQ ID NO: 1.

## Patentansprüche

1. Zusammensetzung zur Verwendung in einer adoptiven Zelltherapie zur Behandlung eines Melanoms, wobei die Zusammensetzung Folgendes umfasst:
(a) ein MYC-Fusionspeptid, umfassend (i) eine Proteintransduktionsdomäne, gegebenenfalls eine TAT-Proteintransduktionsdomänen-Sequenz; (ii) eine MYC-Polypeptid-Sequenz; und
(b) eine oder mehrere primäre Immunzellen, die von einem Spender, der einen Melanom-Tumor aufweist, isoliert wurden, wobei die eine oder die mehreren primären Immunzellen gegen ein melanomspezifisches Antigen reaktiv sind.

2. Zusammensetzung zur Verwendung nach Anspruch 1, wobei das MYC-Fusionspeptid die SEQ ID NR: 1 umfasst.

3. Zusammensetzung zur Verwendung nach einem der Ansprüche 1 - 2, wobei die eine oder die mehreren Immunzellen ein oder mehrere Lymphozyten umfassen, gegebenenfalls eine T-Zelle, eine B-Zelle, eine NK-Zelle oder eine beliebige Kombination davon.

4. Zusammensetzung zur Verwendung nach Anspruch 3, wobei der eine oder die mehreren Lymphozyten einen tumorinfiltrierenden Lymphozyten umfassen oder rekombinant modifiziert vorliegen, um einen modifizierten Rezeptor oder Chimären Antigenrezeptor zu exprimieren.

5. Modifizierte Immunzelle zur Verwendung in einer adoptiven Zelltherapie zur Behandlung eines Melanoms bei einem Individuum, wobei die modifizierte Immunzelle ein MYC-Fusionspeptid umfasst, das (i) eine Proteintransduktionsdomäne, gegebenenfalls eine TAT-Proteintransduktionsdomänen-Sequenz; und (ii) eine MYC-Polypeptid-Sequenz umfasst und gegen ein tumorspezifisches Antigen reaktiv ist.

6. Modifizierte Immunzelle zur Verwendung nach Anspruch 5, wobei das MYC-Fusionspeptid SEQ ID NR: 1 umfasst; und/oder wobei die eine oder die mehreren Immunzellen ein oder mehrere Lymphozyten umfassen, gegebenenfalls eine T-Zelle, eine B-Zelle, eine NK-Zelle oder eine beliebige Kombination davon und wobei gegebenenfalls der eine oder die mehreren Lymphozyten einen tumorinfiltrierenden Lymphozyten umfassen oder rekombinant modifiziert vorliegen, um einen modifizierten Rezeptor oder Chimären Antigenrezeptor zu exprimieren.

7. Modifizierte Immunzelle zur Verwendung nach Anspruch 5 oder 6, wobei die eine oder die mehreren modifizierten Immunzellen von primären Immunzellen stammen, die von dem Empfänger isoliert wurden oder von einem separaten Spender, der denselben Typ Melanom aufweist, isoliert wurden.

8. Modifizierte Immunzelle zur Verwendung nach einem der Ansprüche 5 - 7, wobei die eine oder die mehreren modifizierten Immunzellen durch Inkontaktbringen der primären Immunzellen *in vitro* mit dem MYC-Fusionspeptid nach der Isolierung von dem Spender hergestellt werden.

9. Modifizierte Immunzelle zur Verwendung nach einem der Ansprüche 7 - 8, ferner umfassend das Expandieren der primären Immunzellen *in vitro* vor und nach dem Inkontaktbringen mit dem MYC-Fusionspeptid, wobei die Zellen gegebenenfalls unter Verwendung eines Anti-CD3-Antikörpers, bestrahlten allogenen Feederzellen oder eines exogenen Zytokins expandiert werden, wobei es sich bei dem Zytokin gegebenenfalls um Interleukin-2 handelt.

10. Modifizierte Immunzelle zur Verwendung nach einem der Ansprüche 5 - 9, wobei der (dem) Empfänger:
(i) vor der Verabreichung der einen oder mehreren modifizierten Immunzellen einer Lymphodepletion unterzogen wird;
(ii) vor, während oder im Anschluss an die Verabreichung der einen oder mehreren Immunzellen ein Zytokin verabreicht wird, wobei das Zytokin gegebenenfalls aus der Gruppe bestehend aus Interferon α, Interferon β, Interferon γ, Komplement C5a, IL-2, TNFalpha, CD40L, IL12, IL-23, IL15,IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 und XCL2 ausgewählt wird; und/oder
(iii) ein Mensch oder ein nicht menschliches Tier ist.

11. Modifizierte Immunzelle zur Verwendung nach einem der Ansprüche 5 - 10, wobei das Melanom metastatisch ist.

12. Modifizierte Immunzelle zur Verwendung nach einem der Ansprüche 5 - 11, wobei dem Empfänger eine zusätzliche Krebstherapie verabreicht wurde, die gegebenenfalls unter Chemotherapie, Strahlentherapie, Immuntherapie, monoklonalen Antikörpern, Anti-Krebs-Nukleinsäuren oder -Proteinen, Anti-Krebs-Viren oder - Mikroorganismen und einer beliebigen Kombination davon ausgewählt wird.

13. Verfahren zum Herstellen von modifizierten Immunzellen für eine adoptive Zelltherapie bei Melanom, umfassend das Inkontaktbringen einer oder mehrerer Immunzellen *in vitro* mit einem MYC-Fusionspolypeptid, wobei die Immunzellen von einem Spender sind, der einem oder mehreren Tumor-Antigenen ausgesetzt war und wobei das MYC-Fusionspeptid (i) eine Proteintransduktionsdomäne, gegebenenfalls eine TAT-Proteintransduktionsdomänen-Sequenz; (ii) eine MYC-Polypeptid-Sequenz umfasst und die gegen ein tumorspezifisches Antigen reaktiv sind.

14. Verfahren nach Anspruch 13, wobei die eine oder die mehreren modifizierten Immunzellen von primären Immunzellen stammen, die von einem Individuum isoliert wurden, das Melanom aufweist.

15. Verfahren nach einem der Ansprüche 13 - 14, ferner umfassend das Expandieren der primären Immunzellen *in vitro* vor und nach dem Inkontaktbringen mit dem MYC-Fusionspeptid; und/oder wobei das MYC-Fusionspeptid SEQ ID NR: 1 aufweist.

## Revendications

1. Composition pour une utilisation en thérapie cellulaire adoptive pour le traitement d'un mélanome, la composition comprenant :
(a) un peptide de fusion de MYC, comprenant (i) un domaine de transduction de protéine, éventuellement une séquence de domaine de transduction de protéine TAT ; (ii) une séquence polypeptidique de MYC ; et
(b) une ou plusieurs cellules immunitaires primaires isolées d'un sujet donneur qui a une tumeur mélanome, la ou les cellules immunitaires primaires étant réactives vis-à-vis d'un antigène spécifique de mélanome.

2. Composition pour l'utilisation selon la revendication 1, dans laquelle le peptide de fusion de MYC comprend la SEQ ID NO: 1.

3. Composition pour l'utilisation selon l'une quelconque des revendications 1 à 2, dans laquelle la ou les cellules immunitaires comprennent un ou plusieurs lymphocytes, éventuellement une cellule T, une cellule B, une cellule NK ou toute combinaison de ceux-ci.

4. Composition pour l'utilisation selon la revendication 3, dans laquelle le ou les lymphocytes comprennent un lymphocyte d'infiltration tumorale ou sont modifiés par recombinaison pour exprimer un récepteur modifié ou un récepteur d'antigène chimérique.

5. Cellule immunitaire modifiée pour une utilisation en thérapie cellulaire adoptive pour le traitement d'un mélanome chez un sujet, la cellule immunitaire modifiée comprenant un peptide de fusion de MYC comprenant (i) un domaine de transduction de protéine, éventuellement une séquence de domaine de transduction de protéine TAT ; et (ii) une séquence polypeptidique de MYC, et est réactive vis-à-vis d'un antigène spécifique de tumeur.

6. Cellule immunitaire modifiée pour l'utilisation selon la revendication 5, dans laquelle le peptide de fusion de MYC comprend la SEQ ID NO: 1 ; et/ou la ou les cellules immunitaires comprenant un ou plusieurs lymphocytes, éventuellement une cellule T, une cellule B, une cellule NK ou toute combinaison de ceux-ci, et éventuellement dans laquelle le ou les lymphocytes comprennent un lymphocyte d'infiltration tumorale ou sont modifiés par recombinaison pour exprimer un récepteur modifié ou un récepteur d'antigène chimérique.

7. Cellule immunitaire modifiée pour l'utilisation selon la revendication 5 ou 6, la ou les cellules immunitaires modifiées dérivant de cellules immunitaires primaires isolées du sujet receveur ou isolées d'un sujet donneur distinct ayant le même type de mélanome.

8. Cellule immunitaire modifiée pour l'utilisation selon l'une quelconque des revendications 5 à 7, la ou les cellules immunitaires modifiées étant préparées par mise en contact des cellules immunitaires primaires *in vitro* avec le peptide de fusion de MYC après isolement à partir du sujet donneur.

9. Cellule immunitaire modifiée pour l'utilisation selon l'une quelconque des revendications 7 à 8, comprenant en outre l'expansion des cellules immunitaires primaires *in vitro* avant ou pendant la mise en contact avec le peptide de fusion de MYC, les cellules étant éventuellement expansées par utilisation d'un anticorps anti-CD3, de cellules nourricières irradiées, ou d'une cytokine exogène, la cytokine étant éventuellement une interleukine-2.

10. Cellule immunitaire modifiée pour l'utilisation selon l'une quelconque des revendications 5 à 9, le sujet receveur :
(i) étant lymphodéplété avant administration de la ou des cellules immunitaires modifiées ;
(ii) ayant reçu une administration de cytokine avant, pendant ou après administration de la ou des cellules immunitaires modifiées, la cytokine étant éventuellement choisie dans un groupe consistant en l'interféron α, l'interféron β, l'interféron γ, le complément C5a, l'IL-2, le TNFalpha, CD40L, IL12, IL-23, IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21, CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 et XCL2 ; et/ou (iii) étant un humain ou un animal non humain.

11. Cellule immunitaire modifiée pour l'utilisation selon l'une quelconque des revendications 5 à 10, le mélanome étant métastatique.

12. Cellule immunitaire modifiée pour l'utilisation selon l'une quelconque des revendications 5 à 11, le sujet receveur ayant reçu une administration d'une thérapie anticancéreuse additionnelle, éventuellement choisie parmi une chimiothérapie, une radiothérapie, une immunothérapie, des anticorps monoclonaux, des acides nucléiques ou des protéines anticancéreux, des virus ou microorganismes anticancéreux, et toutes combinaisons de ceux-ci.

13. Procédé de préparation de cellules immunitaires modifiées pour une thérapie cellulaire adoptive pour un mélanome, comprenant la mise en contact d'une ou plusieurs cellules immunitaires *in vitro* avec un polypeptide de fusion de MYC, les cellules immunitaires provenant d'un donneur qui a été exposé à un ou plusieurs antigènes tumoraux, et le peptide de fusion de MYC comprenant (i) un domaine de transduction de protéine, éventuellement une séquence de domaine de transduction de protéine TAT ; (ii) une séquence polypeptidique de MYC, et étant réactives vis-à-vis d'un antigène spécifique de tumeur.

14. Procédé selon la revendication 13, dans lequel la ou les cellules immunitaires modifiées dérivent de cellules immunitaires primaires isolées d'un sujet ayant un mélanome.

15. Procédé selon l'une quelconque des revendications 13 à 14, comprenant en outre l'expansion des cellules immunitaires primaires *in vitro* avant ou pendant la mise en contact avec le peptide de fusion de MYC ; et/ou dans lequel le peptide de fusion de MYC comprend la SEQ ID NO: 1.
